# EUROPEAN PATENT SPECIFICATION

(11) **EP 2 150 249 B1**
(45) Date of publication and mention of the grant of the patent: **24.04.2013**
(21) Application number: 07757059.6
(22) Date of filing: 15.02.2007
(51) Int. Cl.: A61K 31/353, A61K 31/357, A61K 31/36, A61P 25/06

(54) **USE OF BENZO-FUSED HETEROCYCLE SULFAMIDE DERIVATIVES FOR THE TREATMENT OF MIGRAINE**
VERWENDUNG VON BENZO-KONDENSIERTEN HETEROZYKLISCHEN SULFAMID-DERIVATEN ZUR BEHANDLUNG VON MIGRÄNE
UTILISATION DE DÉRIVÉS DE SULFAMIDE À NOYAU HÉTÉROCYCLIQUE BENZOFUSIONNÉ POUR LE TRAITEMENT DE LA MIGRAINE

(30) Priority: 15.02.2006 US 773764 P; 12.02.2007 US 674021
(43) Date of publication of application: 10.02.2010
(73) Proprietor: Janssen Pharmaceutica N.V., 2340 Beerse (BE)
(72) Inventor: SMITH-SWINTOSKY, Virginia L., Hatfield, PA 19440 (US)
(74) Representative: Warner, James Alexander
(86) International application number: PCT/US2007/062241
(87) International publication number: WO 2007/095618

(56) References cited:
- EP-A1- 0 478 954
- WO-A-02/089785
- WO-A-2006/007435
- WO-A-2006/007436
- MAZZOTTA G ET AL: "Antiepileptic drugs in migraine prophylaxis", JOURNAL OF HEADACHE AND PAIN 200411 IT LNKD- DOI:10.1007/S10194-004-0111-8, vol. 5, no. SUPPL. 2, November 2004 (2004-11), pages S67-S70, ISSN: 1129-2369
- PINI L -A ET AL: "Anti-epileptic drugs in the preventive treatment of migraine headache: A brief review", JOURNAL OF HEADACHE AND PAIN 2001 IT LNKD- DOI:10.1007/S101940170041, vol. 2, no. 1, 2001, pages 13-19, ISSN: 1129-2369
- STEVE D WHEELER: "Antiepileptic drug therapy in migraine headache", CURRENT TREATMENT OPTIONS IN NEUROLOGY,, vol. 4, no. 5, 1 September 2002 (2002-09-01), pages 383-394, XP009135110, ISSN: 1092-8480
- MICHAEL A ROGAWSKI & WOLFGANG LÖSCHER: "The neurobiology of antiepileptic drugs", NATURE REVIEWS NEUROSCIENCE,, vol. 5, 1 July 2004 (2004-07-01), pages 553-564, XP009135121,
- ROGAWSKI MICHAEL A ET AL: "The neurobiology of antiepileptic drugs for the treatment of nonepileptic conditions", NATURE MEDICINE, vol. 10, no. 7, July 2004 (2004-07), pages 685-692, ISSN: 1078-8956

## Description

### FIELD OF THE INVENTION

The present invention is directed to the use of benzo-fused heterocycle sulfamide derivatives derivatives for the treatment and / or prevention of migraine.

### BACKGROUND OF THE INVENTION

Migraine is a chronic, episodic and debilitating clinical condition that is diagnosed by the presence of moderate to severe pulsating unilateral headaches lasting between 4 and 72 h. Additionally, the headache is sometimes associated with temporary sensory (photophobia and phonophobia) and/or gastrointestinal (nausea, vomiting) disturbances. Migraine headaches can present without or with aura.

Migraine without aura is defined by at least five attacks fulfilling the following criteria: (a) the headache attacks lasting 4-72 hours with the headache having at least two of the following features: unilateral location, pulsating quality, moderate or severe intensity wit7.5h a direct influence on activities of daily living, and aggravation by walking up stairs or similar routines; (b) during the headache at least one of the following occurs: nausea and/or vomiting, photophobia or phonophobia (Classification and diagnostic criteria for headache disorders, cranial neuralgias and facial pain. Headache Classification Committee of the International Headache Society. Cephalalgia 1988;8 Suppl 7:1-96).

Migraine with aura is defined by at least two attacks accompanied by at least 3 of the 4 following features: (a) one or more fully reversible aura symptoms; (b) at least one aura symptom which develops gradually over more than four minutes or two or more symptoms which occur in succession; (c) no aura symptom that lasts more than 60 minutes; (d) the headache begins prior to, simultaneously with or following the aura, with a free interval between aura and headache of less than about 60 minutes (Classification and diagnostic criteria for headache disorders, cranial neuralgias and facial pain. Headache Classification Committee of the International Headache Society. Cephalalgia 1988;8 Suppl 7:1-96).

The clinical profiles of patients with migraine headaches are represented by migraine without aura (about 70% of migraineurs) and migraine with aura (about 30%). Migraine without aura is also known as common migraine and typically has an average duration of about 18 to 24 hours. Pain is usually unilateral, but it can alternate sides or be bilateral during an attack. Migraine with aura can be associated with visual disturbances and the aura usually develops gradually over 5-20 min and usually lasts less than 60 minutes. Migraine with aura may be sequentially associated with attacks without aura. The most common form of migraine with aura is migraine with typical aura also known as classical migraine. Headache pain commences within 60 minutes of the end of the aura. Other less common types of migraine headaches exist and include, but are not limited to, migraine with prolonged aura which is associated with aura symptoms that last longer than 60 minutes; migraine aura without headache; migraine with acute onset aura; basilar migraine which can be associated with vertigo, gait disturbances and/or loss of consciousness; ophthalmoplegic migraine associated with ocular paralysis, diplopia and ptosis; retinal migraine; and familial hemiplegic migraine associated with hemiparesis or hemiplegia (Migraine. Cognos. Decision Resources, 2000).

Pharmacological interventions for the therapeutic management of migraine can be categorized into two general strategies: preventive approaches and treatments to relieve the pain and associated symptomatology or abortive therapy.

The objective of the preventive (prophylactic) therapy is to reduce the frequency of the migraine attacks, reduce the severity and/or shorten the duration of the attacks. Prophylactic treatments for migraine include anticonvulsants, antidepressants, beta blockers, calcium channel blockers nonsteroidal anti-inflammatory drugs (NSAIDs), and serotonin receptor antagonists. Many of these agents are used off-label in migraine prophylaxis. (Migraine. Cognos. Decision Resources, 2000).

Based on clinical studies, specific agents within the classes of antidepressants and beta-blockers have been shown to have the highest efficacy and the best adverse side effects profile.

Anticonvulsants used in migraine prophylaxis include, but are not limited to, topiramate (Ortho-McNeil's TOPAM™), valproic acid (Abbott's DEPAKENE™), divalproex sodium (Abbott's DEPAKOTE™), and gabapentin (Warner-Lambert's NEURONTIN™).

Antidepressants used in migraine prophylaxis include, but are not limited to, tricyclic antidepressants such as amitriptyline (Schering's ETRAFON™, ICN's LIMBITROL™, Banyu's TRYPTANOL™, Bayer's SAROTEN™, Roche's LAROXYL™, Astra Zeneca's ELAVIR™, and generics), nortriptyline (Novartis' PAMELOR™, and generics), clomipramine (Novartis' ANAFRANIL™, and generics), imipramine (Novartis' TOFRANIL™, and generics), doxepin (Pfizer's SINEQUAN™, and generics); mono-amine oxidase inhibitors such as phenelzine (Parke-Davis' NARDIL™); selective serotonin reuptake inhibitors such as fluoxetine (Eli Lilly's PROZAC™, SARAFEM™ and generics), fluvoxamine (Solvay's LUVOX™), citalopram (Lundbeck's CIPRAMIL™, and Forest's CELEXA™); and selective serotonin noradrenaline reuptake inhibitors such as venlafaxine (Wyeth-Ayerst's EFFEXOR XR™).

Beta blockers used in migraine prophylaxis include, but are not limited to, metoprolol (Astra-Zeneca's TOPROL-XR™, Novartis' LOPRESSOR™, and generics), atenolol (Astra Zeneca's TENORMIN™, TEMORETIC™, and generics), propanolol (Wyeth-Ayerst's INDERAL™, and generics), timolol (Merck, Sharp and Dohme's BLOCADREN, Falcon's TIMOLOL™, and generics), and nadolol (Bristol-Myers Squibb's Monarch's CORGARD™/SOLOFOL™, Dainippon's NADIC™, and generics).

Calcium channel blockers used in migraine prophylaxis include, but are not limited to, verapamil (Knoll's ISOPTIN™, Schwarz's Verelan, Searle's Covera and CALAN™, and generics), Iomerizine (TERRANAS™ from Nippon Organon's), flunarizine (SIBELIUM™, from Janssen Pharmaceutica), diltiazem (Biovail CARDIZEM™, and generics), nimodipine (Bayer, NIMOTOP™, and ESTEVE™), zucapsaicin (Civamide™, from Winston Laboratories), and dotarizine™ (from Mylan/Ferrer).

Nonsteroidal anti-inflammatory drugs used in migraine prophylaxis include, but are not limited to, naproxen (Roche Laboratories' Naprosyn and generics) and ketoprofen (Wyeth-Ayerst's ORUDIS™ and ORUVAIL™ and generics).

Serotonin receptor antagonists used in migraine prophylaxis include, but are not limited to, Pizotifen (Novartis's SANOMIGRAN™/PIZOTYLINE™), methysergide (Novartis' SANSERT™/DESERIL™, and generics), and cyproheptadine (Merck's PERIACTIN™).

Abortive treatments in the management of migraine headache (the relief of the pain and/or associated symptomology of migraine attacks) include analgesics and combinations, antiemetics, ergot derivatives, nonsteroidal anti-inflammatory drugs, and triptans. Neuropeptide antagonists are also been studied. (Migraine. Cognos. Decision Resources, 2000).

Analgesics and combinations (including combinations with other drugs such as antiemetics) for the abortive treatment of migraine include, but are not limited to aspirin, acetaminophen, paracetamol, meperidine, codeine, hydrocodone, Novartis' FIORICET™ or Forests' ESGIC™ or generics (combination of acetaminophen and butalbital and caffeine), FIORINAL™ or generics (combination of aspirin, butalbital and caffeine, Novartis), MIGPRIV™ or generics (combination of aspirin and metoclopromide; Sanofi-Synthelabo), MIDRIN™,/MIDRID™ or generics (combination of acetaminophen and dichloralphenazone; Camick), Sanofi-Synthelabo's PARAMAX™ or Dolorgiet's MIGRAENERTON™ or generics (combination of paracetamol and metoclopramide), Abbott's VICODIN™ or generics (combination of acetaminophen and hydrocodone), STADOL NS™ (butorphanol nasal spray; Bristol-Myers Squibb), Boehringer Ingelheim's LONARID™ or Pfizer's MIGRALEVE™ or generics (combination of paracetamol and codeine).

Antiemetics for the abortive treatment of migraine include but are not limited to, metoclopramide (SmithKline Beecham's MAXOLON™, Robin's REGLAN™, and generics), domperidone (Janssen Pharmaceutica's MOTILIUM™, and generics), prochlorperazine (SmithKline Beecham's COMPAZINE™, and generics), and promethazine (Wyeth-Ayerst's PHENERGAN™/MEPERGAN™, and generics).

Ergot derivatives for the abortive treatment of migraine include, but are not limited to, dihydroergotamine (Novartis DHE-45, MIGRANAL™ nasal spray), ergotamine (Lotus Biochemical's ERGOMAR, and generics), and combination of ergotamine with caffeine (Novartis' CAFERGOT™, Organon's WIGRAINE™, and generics).

Nonsteroidal anti-inflammatory drugs for the abortive treatment of migraine include, but are not limited to, aspirin, ibuprofen, diclofenac (Novartis' VOLTAREN™, and generics), naproxen (Roche's NAPROSYN™, and generics) and ketoprofen (Wyeth-Ayerst's ORUDIS™ and ORUVAIL™, and generics).

Triptans for the abortive treatment of migraine include, but are not limited to, sumatriptan (IMITREX™/IMIGRAN™, Glaxo Welcome), naratriptan AMERGE™ from Glaxo Wellcome), rizatriptan (MAXAL™ from Merck), zolmitriptan (ZOMIG™, from Astra Zeneca), eletriptan (RELPAX™, from Pfizer), frovatriptan (MIGUARD™ from Vernalis/Elan/Menarini), and almotriptan (AXERT™ from Pharmacia).

Neuropeptide antagonists which may be useful in prophylactic as well as abortive therapy of migraine include, but are not limited to, the following agents: calcitonin gene-related peptide antagonist (BIBN 4096 from Boehringer Ingelheim), and substance P antagonists such as dapitant (Aventis's ERISPANT™), lanepitant (Lilly's LY-303870) and FK-888 from Fujisawa.

Drugs for prophylactic treatment of migraine must be taken daily and many are associated with undesired adverse effects. For example, the use of methysergide carries with it the danger of retroperitoneal fibrosis. For nonsteroidal anti-inflammatory drugs the need for high dosages for effectiveness is a drawback. Tricyclic antidepressants are associated with multiple side effects including sedation, weight gain and anticholinergic effects including dry mouth, blurred vision, constipation, cognitive impairment, and urinary retention. Monoamine oxidase inhibitors are often associated with side effects which include orthostatic hypotension, hypertensive crisis, body weight gain, insomnia and sexual dysfunction. Selective serotonin reuptake inhibitors side effects include nausea, diarrhea, constipation, sleep impairment, sexual dysfunction, and anxiety and the risk for serotonin syndrome. Venlafaxine can be associated with unwanted cardiovascular effects, sedation, anticholinergic effects, gastrointestinal disturbances, and sexual dysfunction. Valproic acid side effects include drowsiness, nausea, fatigue, tremor, and weight gain. In many cases it is the side effects that are the cause for noncompliance and self-discontinuation. In addition, it has been estimated that the probability of success with any one of the available prophylactic anti-migraine drugs is about 60-70% (Harrison's Principles of Internal Medicine, eds. Isselbacher et al., McGraw-Hill, Inc., New York, 1994, p/69).

WO 2006/007435 and WO 2006/007436 disclose sulfamate and sulfamide derivatives for the treatment of epilepsy and related disorders.

There remains a need to provide an effective treatment and prevention of migraine

Mazzotta, G.; Floridi, F.; Alberti, A; Sarchielli, P. J Headache Pain, 2004, 5, S67-S70 discloses a review of data in the literature regarding the use of antiepileptic drugs in migraine prophylaxis.

Lupo, L.; Luigi-Alberto, P. J Headache Pain, 2001, 2, 13-19 discloses a review of anti-epileptic drugs in the preventive treatment of migraine headache.

Wheeler, S. D. Current Treatment Options in Neurology, 2002, 4, 383-394 discloses a review of antiepileptic drug therapy in migraine headache.

Löscher, W.; Rogawski, M.A. Nature Reviews: Neuroscience 2004, 5, 553-564 discloses a review of the neurobiology of antiepileptic drugs.

Löscher, W.; Rogawski, M.A. Nature Medicine 2004, 10 (7), 685-692 discloses a review of the neurobiology of antiepileptic drugs for the treatment of nonepileptic conditions.

### SUMMARY OF THE INVENTION

The present invention is directed to a compound of formula (I) or a pharmaceutically acceptable salt thereof for use in a method for the treatment of migraine 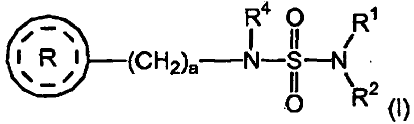 wherein
R¹ and R² are each independently selected from the group consisting of hydrogen and lower alkyl;
R⁴ is selected from the group consisting of hydrogen and lower alkyl;
a is an integer from 1 to 2; 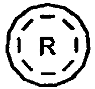 is selected from the group consisting of 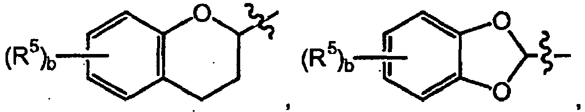 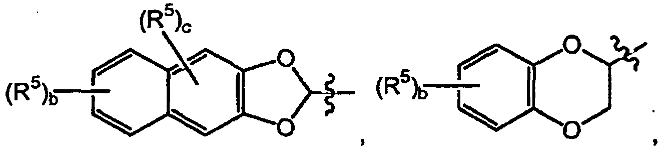 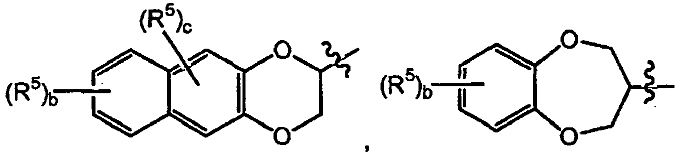 and 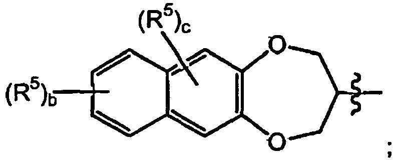 wherein b is an integer from 0 to 4; and wherein c is an integer from 0 to 2;
each R⁵ is independently selected from the group consisting of halogen, lower alkyl and nitro;
provided that when 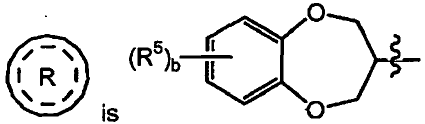 or 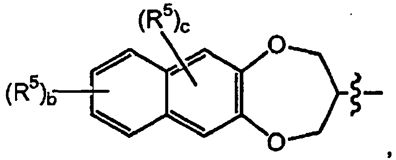 then a is 1

The present invention is further directed to a compound of formula (II) or a pharmaceutically acceptable salt thereof for use in a method for the treatment of migraine.

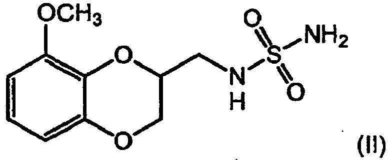

Exemplifying the invention is any of the compounds or pharmaceutical compositions described above for use in a method of treating migraines.

Disclosed herein is a method for treating nausea, vomiting, photophobia and / or phonophobia, preferably nausea, photophobia and / or phonophobia, associated with migraine headaches comprising administering to a subject in need thereof a therapeutically effective amount of a compound of formula (I) or formula (II).

Disclosed herein is a method for the treatment and / or prevention of migraine which comprises co-therapy with a therapeutically effective amount of a compound of formula (I) or formula (II); and an anti-migraine agent, wherein the anti-migraine agent is a prophylactic agent. In another embodiment of the present invention is a method for the treatment and / or prevention of migraine which comprises co-therapy with a therapeutically effective amount of a compound of formula (I) or formula (II); and an anti-migraine agent, wherein the anti-migraine agent is a an abortive agent.

In an embodiment of the present invention, the anti-migraine agent is a triptan. Preferably, the triptan is selected from the group consisting of sumatriptan (IMITREX™/IMIGRAN™, Glaxo Wellcome), naratriptan (AMERGE™ from Glaxo Wellcome), rizatriptan (MAXALT™ from Merck), zolmitriptan (ZOMIG™ from Astra Zeneca), eletriptan (RELPAX™ from Pfizer), frovatriptan (MIGUARD™ from Vernalis/Elan/Menarini), and almotriptan (AXERT™ from Pharmacia).

Disclosed herein is a method for the treatment and/or prevention of migraine which comprises co-therapy with a therapeutically effective amount of a compound of formula (I) or formula (II); and a compound selected from the group consisting of analgesics, antiemetics, ergot derivatives, nonsteroidal anti-inflammatory drugs, triptans, neuropeptide antagonist, anticonvulsants, antidepressants, beta-blockers, calcium channel blockers and serotonin receptor antagonists.

Disclosed herein is a method for the treatment of migraine which comprises co-therapy with a therapeutically effective amount of a compound of formula (I) or formula (II) and a compound selected from the group consisting of analgesics, antiemetics, ergot derivatives, nonsteroidal anti-inflammatory drugs, triptans and neuropeptide antagonists.

Disclosed herein is a method for the prevention of migraine which comprises co-therapy with a therapeutically effective amount of a compound of formula (I) or formula (II) and a compound selected from the group consisting of anticonvulsants, antidepressants, beta-blockers, calcium channel blockers, nonsteroidal anti-inflammatory drugs and serotonin receptor antagonists.

Disclosed herein is a method for the treatment and / or prevention of migraine which comprises co-therapy with a therapeutically effective amount of a compound of formula (I) or formula (II) and a compound selected from the group consisting of antidepressants, beta blockers and triptans.

### DETAILED DESCRIPTION OF THE INVENTION

The present invention is directed to a compound of formula (I) or a pharmaceutically acceptable salt thereof for use in a method for the treatment of migraines 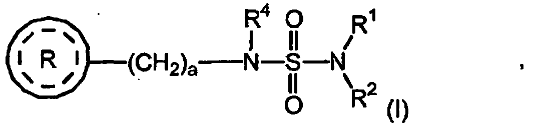 wherein 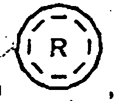 a, R¹, R² and R⁴ are as herein defined. Disclosed herein are methods of reducing the severity and or duration of migraine headaches or episodes. Further disclosed herein are methods of preventing the recurrence of a migraine headache or episode.

Further disclosed are methods for the treatment and / or prevention of migraine comprising administering to a subject in need thereof co-therapy comprising a compound of formula (I) or formula (II) and one or more, preferably one, anti-migraine agent.

As used herein, the term **"migraine"** shall mean a chronic, episodic and debilitating clinical condition that is diagnosed by the presence of moderate to severe pulsating unilateral headaches lasting between 4 and 72 h, which includes migraine without aura and migraine with aura.

As used herein, **"migraine without aura"** shall mean at least five attacks fulfilling the following criteria: (a) the headache attack lasts 4-72 hours with the headache having at least two of the following features: unilateral location, pulsating quality, moderate or severe intensity with direct influence on activities of daily living, and aggravation by walking up stairs or similar routines; and (b) during the headache at least one of the following occurs: nausea and/or vomiting, and photophobia and phonophobia.

As used herein, **"migraine with aura"** shall mean at least two attacks accompanied by at least 3 of the 4 following features: (a) one or more fully reversible aura symptoms; (b) at least one aura symptom which develops gradually over more than four minutes or two or more symptoms which occur in succession; (c) no aura symptom which lasts more than 60 minutes; (d) a headache occurs prior to, simultaneously with or following the aura, with a free interval between aura and headache of less than about 60 minutes.

As used herein, the term **"prevention"** shall include the prevention of migraine attacks (headaches), a decrease in the frequency of migraine attacks (headaches), a decrease in the severity of migraine attacks (headaches) and/or a decrease in the duration of migraine attacks (headaches).

As used herein, the term **"prophylactic agent"** shall mean any pharmaceutical agent which may be used for the prevention or prophylaxis of migraine. Suitable examples include, but are not limited to pharmaceutical agents in the classes of anticonvulsants, antidepressants, beta blockers, calcium channel blockers, nonsteroidal anti-inflammatory drugs (NSAIDs) and serotonin receptor antagonist.

As used herein, the term **"abortive agent"** shall mean any pharmaceutical agent which may be used for the treatment of migraine. Suitable examples include, but are not limited to pharmaceutical agents in the classes of analgesics and combinations, antiemetics, ergot derivatives, nonsteroidal anti-inflammatory drigs (NSAIDs), triptans and neuropeptide antagonists.

As used herein, the term **"anti-migraine agent"** shall include any pharmacological agent which may be used to treat or prevent migraine attacks (i.e. any pharmacological agent which may be used for the treatment or prophylaxis of migraine). Suitable examples include, but are not limited to, pharmacological agents in the classes of anticonvulsants, antidepressants, beta-blockers, calcium channel blockers, nonsteroidal anti-inflammatory agents, serotonin receptor antagonists, serotonin reuptake inhibitors, serotonin noradrenaline reuptake inhibitors, analgesics, antiemetics, ergot derivatives, triptans, neuropeptide antagonists and riboflavin (vitamin B2).

As used herein anticonvulsants includes, but are not limited to, valproic acid (usual daily oral dosage of 10 to 60 mg) (Abbott's DEPAKENE™), divalproex sodium (usual daily oral dosage of 10 to 60 mg) (Abbott's DEPAKOTE™), and gabapentin (usual daily oral dosage of 300 to 1800 mg for adults, with lower dosage levels for children) (Warner-Lambert's NEURONTIN™).

As used herein antidepressants, include but are not limited, to tricyclic antidepressants such as amitriptyline (usual daily oral therapeutic dose range of 150-300 mg) (Schering's ETRAFON™, ICN's LIMBITROL™, Banyu's TRYPTANOL™, Bayer's SAROTEN™, Roche's LAROXYL, Astra Zeneca's ELAVIL™, and generics), nortriptyline (usual daily oral therapeutic dose range of 50-150 mg) (Novartis' PAMELOR™, and generics), clomipramine (usual daily oral therapeutic dose range of 100-250 mg) (Novartis' ANAFRANIL™, and generics), imipramine (usual daily oral therapeutic dose range of 150-300 mg) (Novartis' TOFRANIL™, and generics), doxepin (usual daily oral therapeutic dose range of 150-300 mg) (Pfizer's SINEQUAN™, and generics); mono-amine oxidase inhibitors such as phenelzine (usual daily oral therapeutic dose range of 45-90 mg) (Parke-Davis' NARDIL™); selective serotonin reuptake inhibitors such as fluoxetine (usual daily oral therapeutic dose range of 20-60 mg) (Eli Lilly's PROZAC™, SARAFEM™ and generics), fluvoxamine (usual daily oral therapeutic dose range of 100-300 mg) (Solvay's LUVOX™), citalopram (usual daily oral therapeutic dose range of 20-40 mg) (Lundbeck's CIPRAMIL™, and Forest's CELEXA™); and selective serotonin noradrenaline reuptake inhibitors such as venlafaxine (usual daily oral therapeutic dose range of 125-375 mg) (Wyeth-Ayerst's EFFEXOR™).

Beta blockers include, but are not limited to, metoprolol (usual daily oral therapeutic dose of about 200 mg) (Astra-Zeneca's TOPOL-XL™, Novartis' LOPRESSOR™, and generics), atenolol (usual daily oral therapeutic dose of about 100 mg) (Astra Zeneca's TENORMIN™ and TEMORETIC™, and generics), propanolol (usual daily oral therapeutic dose of about 160 mg) (Wyeth-Ayerst's INDERAL™, and generics), timolol (usual daily oral therapeutic dose of about 20 mg) (Merck, Sharp and Dohme's BLOCADREN™, Falcon's TIMOLOL™, and generics), and nadolol (usual daily oral therapeutic dose of about 160 mg) (Bristol-Myers Squibb's-Monarch's CORGARD™/SOLGOL™, Dainippon's NADIC™, and generics).

Calcium channel blockers include, but are not limited to, verapamil (usual daily oral dosage of 120 to 480 mg) (Knoll's ISOPTIN™, Schwarz's Verelan, Searle's Covera and CALAN™, and generics), Iomerizine (TERRANAS™ from Nippon Organon's), flunarizine (SIBELIUM from Janssen Pharmaceutica), diltiazem (usual daily oral dosage of 120 to 360 mg) (Biovail CARDIZEM™, and generics), nimodipine (usual daily oral dosage of 60 to 240 mg) (Bayer, NIMOTOP™ and ESTEVE™) zucapsaicin (Civamide from Winston Laboratories), and dotarizine (from Mylan/Ferrer).

Nonsteroidal anti-inflammatory drugs include, but are not limited to, aspirin, ibuprofen, diclofenac (usual daily oral dosage of 50 to 200 mg) (Novartis' VOLTAREN™, and generics), naproxen (usual daily oral dosage of 500 to 1000 mg) (Roche's NAPROSYN™, and generics) and ketoprofen (usual daily oral dosage of 150 to 300 mg) (Wyeth-Ayerst's ORUDIS™ and ORUVAIL™, and generics).

As used herein, serotonin receptor antagonists include, but are not limited to, pizotifen (Novartis's SANOMIGRAN™/PIZOTYLlNE™), methysergide (Novartis' SANSERT™/DESERIL™, and generics), and cyproheptadine (usual daily oral dosage of 4 to 20 mg) (Merck's PERIACTIN™).

Analgesics and combinations (including combinations with other drugs such as antiemetics) include, but are not limited to aspirin, acetaminophen, paracetamol, meperidine, codeine, hydrocodone, Novartis' FIORICET™ or Forests' ESGIC™, or generics (combination of acetaminophen and butalbital and caffeine), FIORINAL™ or generics (combination of aspirin, butalbital and caffeine, Novartis), MIGPRIV™ or generics (combination of aspirin and metoclopromide; Sanofi-Synthelabo), MIDRIN™/MIDRID™ or generics (combination of acetaminophen and dichloralphenazone; Camick), Sanofi-Synthelabo's PARAMAX™ or Dolorgiet's MIGRAENERTON™ or generics (combination of paracetamol and metoclopramide), Abbott's VICODIN™ or generics (combination of acetaminophen and hydrocodone), STADOL NS™ (butorphanol nasal spray; Bristol-Myers Squibb), Boehringer Ingelheim's LONARID™ or Pfizer's MIGRALEVE™ or generics (combination of paracetamol and codeine).

As used herein, antiemetics include, but are not limited to, metoclopramide (usual oral dosage of 10 to 15 mg q.i.d.) (SmithKline Beecham's MAXOLON™, Robin's REGLAN™, and generics), domperidone (Janssen Pharmaceutica's MOTILIUM™, and generics), prochlorperazine (usual oral dosage of 5 to 20 mg q.i.d.) (SmithKline Beecham's COMPAZINE™, and generics) and promethazine (usual oral dosage of 12.5 to 50 mg) (Wyeth-Ayerst's PHENERGAN™/MEPERGAN™ and generics).

Ergot derivatives include, but are not limited to, dihydroergotamine (Novartis DHE-45, MIGRANAL™ nasal spray), ergotamine (Lotus Biochemical's ERGOMAR^{™}, and generics), and combination of ergotamine with caffeine (Novartis CAFERGOT, Organon's WIGRAINE^{™}, and generics).

Triptans that include, but are not limited to, sumatriptan (usual therapeutic oral dose of about 50 mg) (IMITREX^{™}/IMIGRAN^{™}, Glaxo Wellcome), naratriptan (usual therapeutic oral dose of about 2.5 mg) (AMERGE^{™}, Glaxo Wellcome), rizatriptan (usual therapeutic oral dose of 5-10 mg) (MAXALT^{™}, Merck), zolmitriptan (usual therapeutic oral dose of about 2.5 mg) (ZOMIG^{™}, Astra Zeneca), and newer triptans including but not limited to eletriptan (RELPAX^{™}, Pfizer), frovatriptan (MIGUARD^{™}, Vernalis/Elan/Menarini), and almotriptan (AXERT^{™} from Pharmacia).

As used herein, neuropeptide antagonists include but are not limited to the following agents: calcitonin gene-related peptide antagonist (BIBN 4096 from Boehringer Ingelheim), and substance P antagonists such as dapitant (Aventis's ERIsPANT^{™}), lanepitant (Lilly's LY-303870) and FK-888 from Fujisawa.

Therapeutically effective dosage levels and dosage regimens for anticonvulsants, antidepressants, beta-blockers, calcium channel blockers, nonsteroidal anti-inflammatory drugs, serotonin receptor antagonists, analgesics, antiemetics, ergot derivatives, triptans, neuropeptide antagonists, and other pharmaceutical agents disclosed herein, may be readily determined by one of ordinary skill in the art. For example, therapeutic dosage amounts and regimens for pharmaceutical agents approved for sale are publicly available, for example as listed on packaging labels, in standard dosage guidelines, in standard dosage references such as the Physician's Desk Reference (Medical Economics Company or online at http://www.pdrel.com) and other sources.

As used herein, the term "subject" refers to an animal, preferably a mammal, most preferably a human, who is the object of treatment, observation or experiment.

The term "**therapeutically effective amount**" as used herein, means that amount of active compound or pharmaceutical agent that elicits the biological or medicinal response in a tissue system, animal or human that is being sought by a researcher, veterinarian, medical doctor or other clinician, which includes prevention and/or alleviation of the symptoms of the disease or disorder being treated. Wherein the present invention is directed to co-therapy comprising administration of one or more compound(s) of formula (I) and one or more anti-migraine agent(s), "therapeutically effective amount" shall mean that amount of the combination of agents taken together so that the combined effect elicits the desired biological or medicinal response. For example, the therapeutically effective amount of co-therapy comprising administration of a compound of formula (I) or formula (II) and an anti-migraine agent would be the amount of the compound of formula (I) or formula (II) and the amount of the anti-migraine agent that when taken together or sequentially have a combined effect that is therapeutically effective. Further, it will be recognized by one skilled in the art that in the case of co-therapy with a therapeutically effective amount, as in the example above, the amount of the compound of formula (I), formula (II) and/or the amount of the anti-migraine agent individually may or may not be therapeutically effective.

As used herein, the term **"co-therapy"** shall mean treatment of a subject in need thereof by administering one or more compounds of formula (I) or formula (II) with one or more anti-migraine agents, wherein the compound(s) of formula (I) or formula (II) and the anti-migraine agent(s) are administered by any suitable means, simultaneously, sequentially, separately or in a single pharmaceutical formulation. Where the compound(s) of formula (I) or formula (II) and the anti-migraine agent(s) are administered in separate dosage forms, the number of dosages administered per day for each compound may be the same or different. The compound(s) of formula (I) or formula (II) and the anti-migraine agent(s) may be administered via the same or different routes of administration. Examples of suitable methods of administration include, but are not limited to, oral, intravenous (iv), intramuscular (im), subcutaneous (sc), transdermal, and rectal. Compounds may also be administered directly to the nervous system including, but not limited to, intracerebral, intraventricular, intracerebroventricular, intrathecal, intracisternal, intraspinal and / or peri-spinal routes of administration by delivery via intracranial or intravertebral needles and / or catheters with or without pump devices. The compound(s) of formula (I) and the anti-migraine agent(s) may be administered according to simultaneous or alternating regimens, at the same or different times during the course of the therapy, concurrently in divided or single forms.

In an embodiment of the present invention R¹ is selected from the group consisting of hydrogen and methyl. In another embodiment of the present invention R² is selected from the group consisting of hydrogen and methyl. In yet another embodiment of the present invention R¹ and R² are each hydrogen or R¹ and R² are each methyl.

In an embodiment of the present invention -(CH₂)ₐ- is selected from the group consisting of -CH₂- and -CH₂-CH₂-. In another embodiment of the present invention -(CH₂)ₐ- is -CH₂-.

In an embodiment of the present R⁴ is selected from the group consisting of hydrogen and methyl, preferably, R⁴ is hydrogen.

In an embodiment of the present invention a is 1.

In an embodiment of the present invention b is an integer from 0 to 2. In another embodiment of the present invention c is an integer from 0 to 2. In another embodiment of the present invention b is an integer from 0 to 1. In another embodiment of the present invention c is an integer from 0 to 1. In yet another embodiment of the present invention the sum of b and c is an integer form 0 to 2, preferably an integer form 0 to 1. In yet another embodiment of the present invention b is an integer from 0 to 2 and c is 0.

In an embodiment of the present invention, 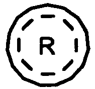 is selected from the group consisting of 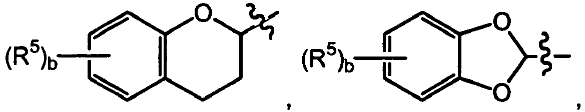 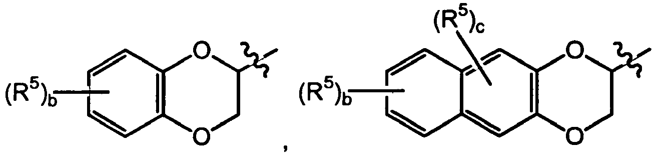 and 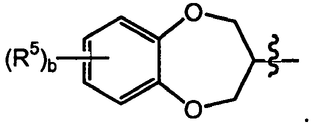 In another embodiment of the present invention, 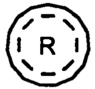 is selected from the group consisting of

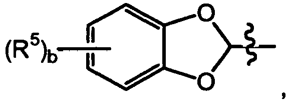

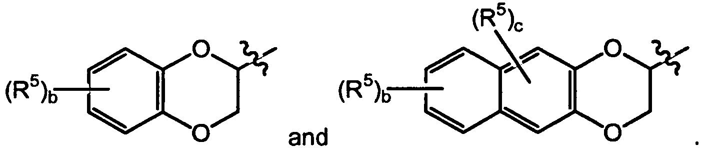

In an embodiment of the present invention, 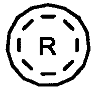 is selected from the group consisting of 2-(2,3-dihydro-benzo[1,4]dioxinyl), 2-(benzo[1,3]dioxolyl), 3-(3,4-dihydro-benzo[1,4]dioxepinyl), 2-(6-chloro-2,3-dihydro-benzo[1,4]dioxinyl), 2-(6-fluoro-2,3-dihydro-benzo[1,4]dioxinyl), 2-(chromanyl), 2-(5-fluoro-2,3-dihydro-benzo[1,4]dioxinyl), 2-(7-chloro-2,3-dihydro-benzo[1,4]dioxinyl), 2-(6-chloro-benzo[1,3]dioxolyl), 2-(7-nitro-2,3-dihydro-benzo[1,4]dioxinyl), 2-(7-methyl-2,3-dihydro-benzo[1,4]dioxinyl), 2-(5-chloro-2,3-dihydro-benzo[1,4]dioxinyl), 2-(6-bromo-2,3-dihydro-benzo[1,4]dioxinyl), 2-(6,7-dichloro-2,3-dihydro-benzo[1,4]dioxinyl), 2-(8-chloro-2,3-dihydro-benzo[1,4]dioxinyl), 2-(2,3-dihydro-naphtho[2,3-b][1,4]dioxinyl) and 2-(4-methyl-benzo[1,3]dioxolyl).

In another embodiment of the present invention, 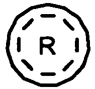 is selected from the group consisting 2-(benzo[1,3]dioxolyl), 2-(2,3-dihydro-benzo[1,4]dioxinyl), 2-(6-chloro-2,3-dihydro-benzo[1,4]dioxinyl), 2-(7-chloro-2,3-dihydro-benzo[1,4]dioxinyl), 2-(7-methyl-2,3-dihydro-benzo[1,4]dioxinyl), 2-(6-bromo-2,3-dihydro-benzo[1,4]dioxinyl) and 2-(6,7-dichloro-2,3-dihydro-benzo[1,4]dioxinyl). In another embodiment of the present invention, 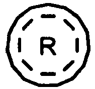 is selected from the group consisting of 2-(2,3-dihydro-benzo[1,4]dioxinyl), 2-(7-methyl-2,3-dihydro-benzo[1,4]dioxinyl) and 2-(6-bromo-2,3-dihydro-benzo[1,4]dioxinyl).

In an embodiment of the present invention R⁵ is selected from the group consisting of halogen and lower alkyl. In another embodiment of the present invention R⁵ is selected from chloro, fluoro, bromo and methyl.

In an embodiment of the present invention, the stereo-center on the compound of formula (I) is in the S-configuration. In another embodiment of the present invention, the stereo-center on the compound of formula (I) is in the R-configuration.

In an embodiment of the present invention the compound of formula (I) is present as an enantiomerically enriched mixture, wherein the % enantiomeric enrichment (% ee) is greater than about 75%, preferably greater than about 90%, more preferably greater than about 95%, most preferably greater than about 98%.

Additional embodiments of the present invention, include those wherein the substituents selected for one or more of the variables defined herein (i.e. R¹, R², R³, R⁴, X-Y and A) are independently selected to be any individual substituent or any subset of substituents selected from the complete list as defined herein.

Representative compounds for use of the present invention, are as listed in Tables 1 below. Additional compounds of the present invention are as listed in Table 3. In Tables 1 and 2 below, the column headed "stereo" defines the stereo-configuration at the carbon atom of the heterocycle attached at the starred bond. Where no designation is listed, the compound was prepared as a mixture of stereo-configurations. Where an "R" or "S" designation is listed, the stereo-configuration was based on the enantiomerically enriched starting material.

**Table 1: Representative Compounds of Formula (I)**

| | | | | | | |
|---|---|---|---|---|---|---|
| 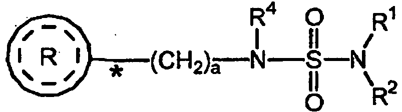 | | | | | | |

| **ID No.** | 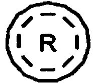 | **Stereo** | **(CH₂)ₐ** | **NR⁴** | **R¹** | **R²** |
|---|---|---|---|---|---|---|
| 1 | 2-(2,3-dihydro-benzo[1,4]dioxinyl) | | CH₂ | NH | H | H |
| 2 | 2-(benzo[1,3]dioxolyl) | | CH₂ | NH | H | H |
| 3 | 3-(3,4-dihydro-2H-benzo[1,4]dioxepinyl) | | CH₂ | NH | H | H |
| 4 | 2-(2,3-dihydro-benzo[1,4]dioxinyl) | S | CH₂ | NH | H | H |
| 5 | 2-(2,3-dihydro-benzo[1,4]dioxinyl) | R | CH₂ | NH | H | H |
| 6 | 2-(2,3-dihydro-benzo[1,4]dioxinyl) | | CH₂ | NH | methyl | methyl |
| 7 | 2-(2,3-dihydro-benzo[1,4]dioxinyl) | | CH₂ | N(CH₃) | H | H |
| 8 | 2-(6-chloro-2,3-dihydro-benzo[1,4]dioxinyl) | S | CH₂ | NH | H | H |
| 9 | 2-(6-fluoro-2,3-dihydro-benzo[1,4]dioxinyl) | S | CH₂ | NH | H | H |
| 10 | 2-(chromanyl) | | CH₂ | NH | H | H |
| 13 | 2-(5-fluoro-2,3-dihydro-benzo[1,4]dioxinyl) | S | CH₂ | NH | H | H |
| 14 | 2-(7-chloro-2,3-dihydro-benzo[1,4]dioxinyl) | S | CH₂ | NH | H | H |
| 15 | 2-(6-chloro-benzo[1,3]dioxolyl) | | CH₂ | NH | H | H |
| 16 | 2-(2,3-dihydro-benzo[1,4]dioxinyl) | | CH₂CH₂ | NH | H | H |
| 18 | 2-(7-nitro-2,3-dihydro-benzo[1,4]dioxinyl) | S | CH₂ | NH | H | H |
| 19 | 2-(7-methyl-2,3-dihydro-benzo[1,4]dioxinyl) | S | CH₂ | NH | H | H |
| 20 | 2-(5-chloro-2,3-dihydro-benzo[1,4]dioxinyl) | S | CH₂ | NH | H | H |
| 22 | 2-(8-methoxy-2,3-dihydro-benzo[1,4]dioxinyl) | S | CH₂ | NH | H | H |
| 24 | 2-(6-bromo-2,3-dihydro-benzo[1,4]dioxinyl) | S | CH₂ | NH | H | H |
| 29 | 2-(6,7-dichloro-2,3-dihydro-benzo[1,4]dioxinyl) | S | CH₂ | NH | H | H |
| 30 | 2-(8-chloro-2,3-dihydro-benzo[1,4]dioxinyl) | S | CH₂ | NH | H | H |
| 33 | 2-(2,3-dihydro-naphtho[2,3-b][1,4]dioxinyl) | S | CH₂ | NH | H | H |
| 35 | 2-(4-methyl-benzo[1,3]dioxolyl) | | CH₂ | NH | H | H |

**Table 2: Additional Compounds of the Present Invention**

| | | | | | | |
|---|---|---|---|---|---|---|
| 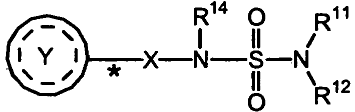 | | | | | | |

| **ID No.** | 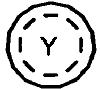 | **Stereo** | **X** | **NR¹⁴** | **R¹¹** | **R¹²** |
|---|---|---|---|---|---|---|
| 23 | 2-(5-methoxy-2,3-dihydro-benzo[1,4]dioxinyl) | S | CH₂ | NH | H | H |
| 26 | 2-(6-methylcarbonyl-2,3-dihydro-benzo[1,4]dioxinyl) | S | CH₂ | NH | H | H |
| 32 | 2-(6-methoxycarbonyl-2,3-dihydro-benzo[1,4]dioxinyl) | S | CH₂ | NH | H | H |
| 34 | 2-(6-hydroxymethyl-2,3-dihydro-benzo[1,4]dioxinyl) | S | CH₂ | NH | H | H |
| 36 | 2-(7-amino-2,3-dihydro-benzo[1,4]dioxinyl) | S | CH₂ | NH | H | H |

As used herein, unless otherwise noted, **"halogen"** shall mean chlorine, bromine, fluorine and iodine.

As used herein, unless otherwise noted, the term **"alkyl"** whether used alone or as part of a substituent group, includes straight and branched chains. For example, alkyl radicals include methyl, ethyl, propyl, isopropyl, butyl, isobutyl, sec-butyl, t-butyl, pentyl and the like. Unless otherwise noted, **"lower"** when used with alkyl means a carbon chain composition of 1-4 carbon atoms.

As used herein, unless otherwise noted, **"alkoxy"** shall denote an oxygen ether radical of the above described straight or branched chain alkyl groups. For example, methoxy, ethoxy, n-propoxy, sec-butoxy, t-butoxy, n-hexyloxy and the like.

As used herein, the notation "*" shall denote the presence of a stereogenic center.

When a particular group is **"substituted"** (e.g., alkyl, aryl, etc.), that group may have one or more substituents, preferably from one to five substituents, more preferably from one to three substituents, most preferably from one to two substituents, independently selected from the list of substituents.

With reference to substituents, the term **"independently"** means that when more than one of such substituents is possible, such substituents may be the same or different from each other.

Under standard nomenclature used throughout this disclosure, the terminal portion of the designated side chain is described first, followed by the adjacent functionality toward the point of attachment. Thus, for example, a **"phenyl-alkyl-amino-carbonyl-alkyl"** substituent refers to a group of the formula

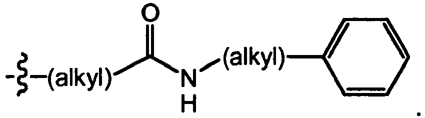

Abbreviations used in the specification, particularly the Schemes and Examples, are as follows:
- DCC: = Dicyclohexyl Carbodiimide
- DCE: = Dichloroethane
- DCM: = Dichloromethane
- DIPEA or DIEA: = Diisopropylethylamine
- DMF: = N,N-Dimethylformamide
- DMSO: = Dimethylsulfoxide
- EDC: = Ethylcarbodiimide
- Et₃N or TEA: = Triethylamine
- Et₂O: = Diethyl ether
- EA or EtOAc: = Ethyl acetate
- EtOH: = Ethanol
- IPA: = 2-propanol
- Hept: = Heptane
- HOBT: = 1-Hydroxybenzotriazole
- HPLC: = High Pressure Liquid Chromatography
- LAH: = Lithium Aluminum Hydride
- M or MeOH: = Methanol
- NMR: = Nuclear Magnetic Resonance
- Pd-C: = Palladium on Carbon Catalyst
- RP HPLC: = Reverse Phase High Pressure Liquid Chromatography
- RT or rt: = Room temperature
- TEA: = Triethylamine
- TFA: = Trifluoroacetic Acid
- THF: = Tetrahydrofuran
- TLC: = Thin Layer Chromatography

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

For use in medicine, the salts of the compounds of this invention refer to non-toxic **"pharmaceutically acceptable salts."** Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following:
acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparation of pharmaceutically acceptable salts include the following:
acids including acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydrocy-ethanesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotine acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and
bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1 H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

Compounds of formula (I) may be prepared according to the process outlined in Scheme 1.

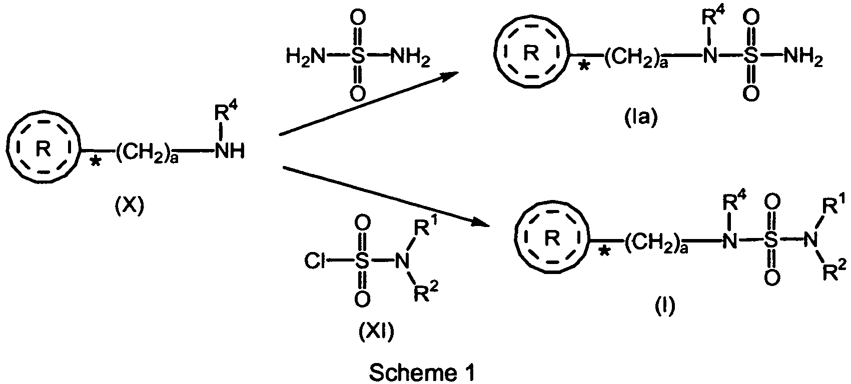

Accordingly, a suitably substituted compound of formula (X), a known compound or compound prepared by known methods, is reacted with sulfamide, a known compound, preferably wherein the sulfamide is present in an amount in the range of about 2 to about 5 equivalents, in an organic solvent such as THF, dioxane, and the like, preferably at an elevated temperature in the range of about 50°C to about 100°C, more preferably at about reflux temperature, to yield the corresponding compound of formula (Ia).

Alternatively, a suitably substituted compound of formula (X), a known compound or compound prepared by known methods, is reacted with a suitably substituted compound of formula (XI), a known compound or compound prepared by known methods, in the presence of a base such as TEA, DIPEA, pyridine, and the like, in an organic solvent such as DMF, DMSO, and the like, to yield the corresponding compound of formula (I).

Compounds of formula (X) wherein 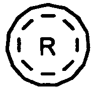 is 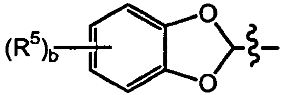 may be prepared according to the process outlined in Scheme 2.

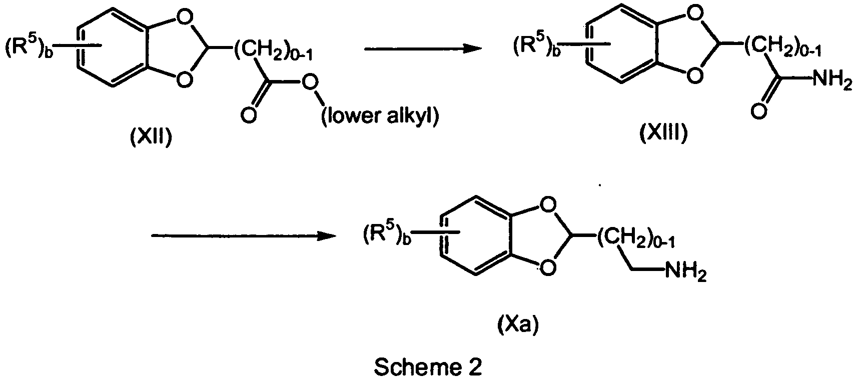

Accordingly, a suitably substituted compound of formula (XII), a known compound or compound prepared by known method (for example as described in Scheme 3 above) is reacted with NH₄OH, a known compound, optionally in an organic solvent such as acetonitrile, and the like, to yield the corresponding compound of formula (XIII).

The compound of formula (XIII) is reacted with a suitably selected reducing agent, such as LAH, and the like, and the like, in an organic solvent such as THF, diethyl ether, and the like, to yield the corresponding compound of formula (Xa).

Compounds of formula (X) wherein 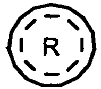 is selected from 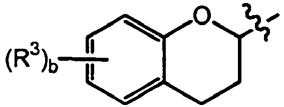 may be prepared according to the process outlined in Scheme 3.

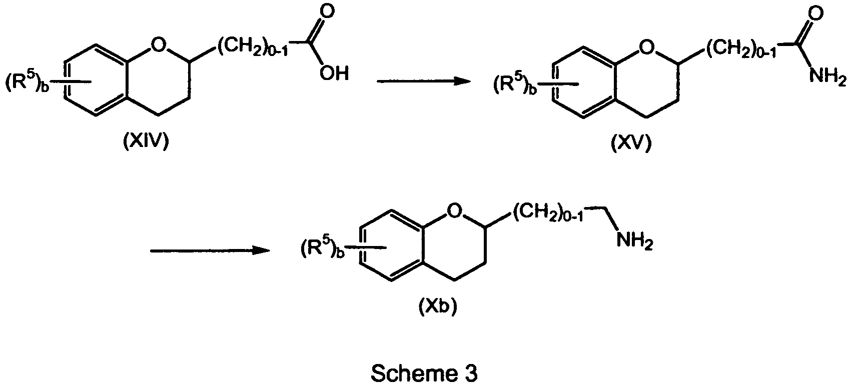

Accordingly, a suitably substituted compound of formula (XIV), a known compound or compound prepared by known methods, is reacted with NH₄OH, in the presence of a coupling agent such as DCC, and the like, optionally in an organic solvent such as acetonitrile, and the like, to yield the corresponding compound of formula (XV).

The compound of formula (XV) is reacted with a suitably selected reducing agent, such as LAH, and the like, in an organic solvent such as THF, diethyl ether, and the like, to yield the corresponding compound of formula (Xb).

Compounds of formula (X) wherein 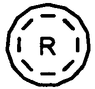 is selected from 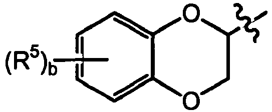 and wherein a is 2, may be prepared according to the process outlined in Scheme 4.

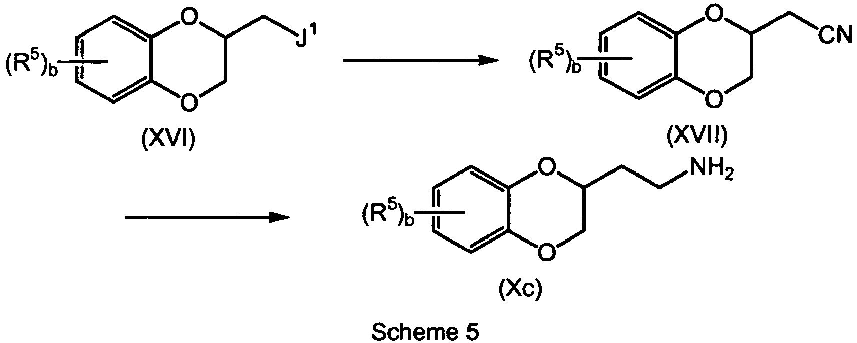

Accordingly, a suitably substituted compound of formula (XVI) wherein J¹ is a suitable leaving group such as Br, Cl, I, tosyl, mesyl, triflyl, and the like, a known compound or compound prepared by known methods (for example, by activating the corresponding compound wherein J¹ is OH), is reacted with a cyanide such as potassium cyanide, sodium cyanide, and the like, in an organic solvent such as DMSO, DMF, THF, and the like, to yield the corresponding compound of formula (XVII).

The compound of formula (XVII) is reduced according to known methods, for example by reacting with a suitable reducing agent such as LAH, borane, and the like, to yield the corresponding compound of formula (Xc).

Compounds of formula (X) wherein 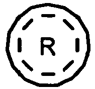 is selected from 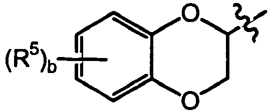 and wherein a is 1, may be prepared according to the process outlined in Scheme 5.

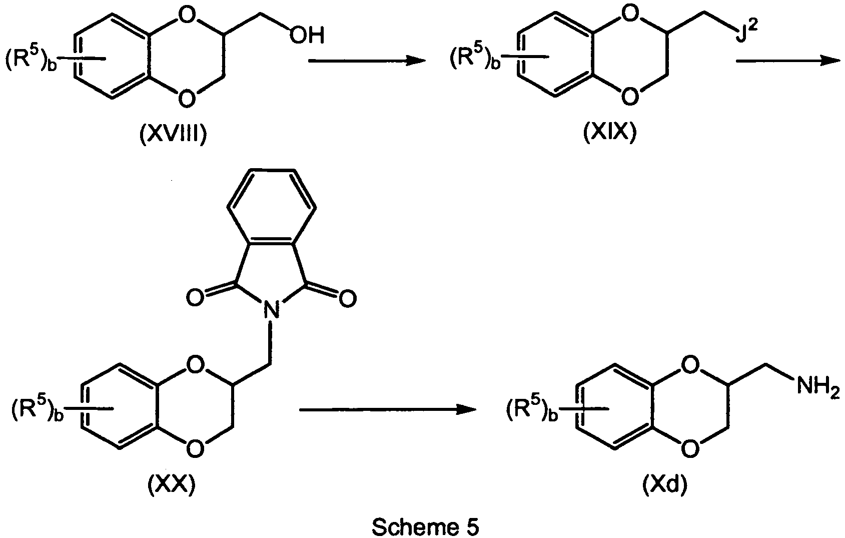

Accordingly, a suitably substituted compound of formula (XVIII), a known compound or compound prepared by known methods is activated, according to known method, to yield the corresponding compound of formula (XIX), wherein J² is a suitable leaving group, such tosylate, Cl, Br, I, mesylate, triflate, and the like.

The compound of formula (XIX) is reacted with a phthalimide salt such as potassium phthlimide, sodium phthalimide, and the like, in an organic solvent such as DMF, DMSO, acetonitrile, and the like, preferably, at an elevated temperature in the range of from 50°C to about 200°C, more preferably, at about reflux temperature, to yield the corresponding compound of formula (XX).

The compound of formula (XX) is reacted with N₂H₄, a known compound, in an organic solvent such as ethanol, methanol, and the like, preferably, at an elevated temperature in the range of from about 50°C to about 100°C, more preferably, at about reflux temperature, and the like, to yield the corresponding compound of formula (Xd).

One skilled in the art will recognize that compounds of formula (X) wherein 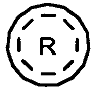 is selected from 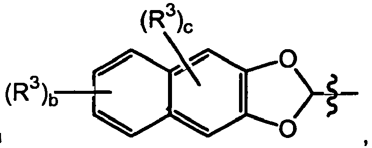 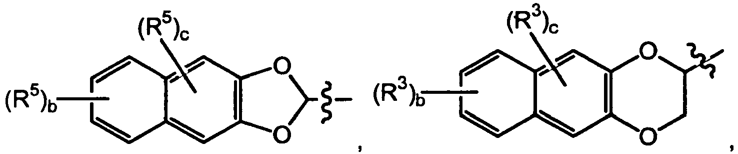 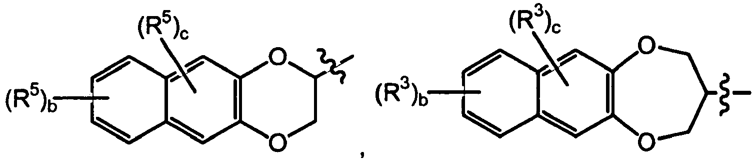 or 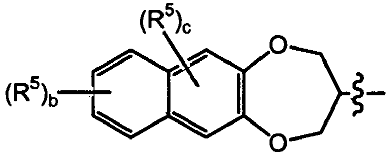 may be similarly prepared according to known methods or for example, according to the processes outlined in Schemes 2 through 5 above, by selecting and substituting the corresponding naphthyl-fused compounds for the benzo-fused starting materials.

One skilled in the art will further recognize that wherein a single enantiomer (or a mixture of enantiomers wherein one enantiomer is enriched) of a compound of formula (X) is desired, the above processes as described in Schemes 1 through 5 may be applied by substituting the corresponding single enantiomer (or mixture of enantiomers wherein one enantiomer is enriched) for the appropriate starting material.

One skilled in the art will recognize that wherein a reaction step of the present invention may be carried out in a variety of solvents or solvent systems, said reaction step may also be carried out in a mixture of the suitable solvents or solvent systems.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

Disclosed herein are pharmaceutical compositions containing one or more compounds of formula (I) with a pharmaceutically acceptable carrier. Pharmaceutical compositions containing one or more of the compounds as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, favoring agents, preservatives, stabilizers, coloring agents and the like, for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare pharmaceutical compositions, one or more compounds of the present invention as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 0.1-1000 mg and may be given at a dosage of from about 0.01-150.0 mg/kg/day, preferably from about 0.1 to 100 mg/kg/day, more preferably from about 0.5-50 mg/kg/day, more preferably from about 1.0-25.0 mg/kg/day or any range therein. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to about 1000 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

The compounds of the present invention may also be used in a pharmaceutical composition comprising any of the compounds as defined herein and a pharmaceutically acceptable carrier. The pharmaceutical composition may contain between about 0.1 mg and 1000 mg, preferably about 50 to 500 mg, of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methylcellulose and the like. For parenteral administration, sterile suspensions and solutions are desired. Isotonic preparations which generally contain suitable preservatives are employed when intravenous administration is desired.

Compounds of this invention may be administered in any of the foregoing compositions and according to dosage regimens established in the art whenever treatment or prevention of migraine is required.

The daily dosage of the products may be varied over a wide range from 0.01 to 150 mg / kg per adult human per day. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250, 500 and 1000 milligrams of the active ingredient for the symptomatic adjustment of the dosage to the patient to be treated. An effective amount of the drug is ordinarily supplied at a dosage level of from about 0.01 mg/kg to about 1500 mg/kg of body weight per day. Preferably, the range is from about 0.1 to about 100.0 mg/kg of body weight per day, more preferably, from about 0.5 mg/kg to about 50 mg/kg, more preferably, from about 1.0 to about 25.0 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

One skilled in the art will recognize that, both *in vivo* and *in vitro* trials using suitable, known and generally accepted cell and / or animal models are predictive of the ability of a test compound to treat or prevent a given disorder.

One skilled in the art will further recognize that human clinical trails including first-in-human, dose ranging and efficacy trials, in healthy patients and / or those suffering from a given disorder, may be completed according to methods well known in the clinical and medical arts.

The following Examples are set forth to aid in the understanding of the invention, and are not intended and should not be construed to limit in any way the invention set forth in the claims which follow thereafter.

### Example 1

### ((3,4-Dihydro-2H-benzo[b][1,4]dioxepin-3-yl)methyl)sulfamide (Compound #3)

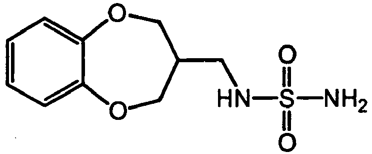

Catechol (5.09 g, 46.2 mmol) and potassium carbonate were combined in acetonitrile and heated to reflux for one hour. 2-Chloromethyl-3-chloro-1-propene (5.78 g, 46.2 mmol) was added and the reaction was continued at reflux for 24 hours. The solution was cooled to room temperature and filtered. The filtrate was evaporated and the residue was diluted with water and extracted with diethyl ether (3 x). The combined organic solution was dried over MgSO₄ and concentrated. Chromatography (2% ethyl ether in hexane) yielded 3-methylene-3,4-dihydro-2H-benzo[b][1,4]dioxepine as a colorless oil.
MS (ESI): 163.2 (M+H⁺)
¹H NMR (300 MHz, CDCl₃), δ: 6.94 (m, 4H), 5.07 (s, 2H), 4.76 (s, 4H).

3-Methylene-3,4-dihydro-2H-benzo[b][1,4]dioxepine (5.00 g, 30.8 mmol) was dissolved in dry THF (100 mL). Borane-THF (1.0 M in THF, 10.3 mL) was added at 0°C. The reaction was stirred at RT for 5 hours. Aminosulfonic acid (6.97 g, 61.6 mmol) was added. The reaction was heated to reflux overnight. The reaction was cooled to room temperature and aqueous sodium hydroxide (3.0 M, 100 mL) was added. The solution was extracted with ethyl acetate (3 x 100 mL). The combined organic solution was dried over MgSO₄. The solution was concentrated under vacuum and purified by chromatography (2% to 8% methanol in dichloromethane) to yield ((3,4-dihydro-2H-benzo[b][1,4]dioxepin-3-yl)methyl)amine as a colorless oil.
MS (ESI): 180.1 (M+H⁺)
¹H NMR (300 MHz, DMSO), δ: 6.92 (m, 4H), 4.21 (m, 2H), 4.07 (m, 2H), 3.33 (broad, 2H), 3.16 (d, *J* = 4 Hz, 1 H), 2.72 (d, *J* = 4 Hz, 1H), 2.30 (m, 1H).
((3,4-Dihydro-2H-benzo[b][1,4]dioxepin-3-yl)methyl)amine (2.90 g, 16.2 mmol) and sulfamide (3.11 g, 32.4 mmol) were combined in dry dioxane (60 ml) and heated to reflux overnight. Chloroform was added and the precipitate was removed by filtration. The filtrate was concentrated under vacuum and purified by chromatography (2% to 8% acetone in dichloromethane) to yield the title compound as an off-white solid.
258.8 (M+H⁺)
¹H NMR (300 MHz, DMSO), δ: 6.92 (m, 4H), 6.71 (broad, 1H), 6.59 (broad, 2H), 4.19 (m, 2H), 4.04 (m, 2H), 3.00 (m, 2H), 2.39 (m, 1H).

### Example 2

### N-(2,3-Dihydro-benzo[1,4]dioxin-2-ylmethyl)-sulfamide (Compound #1)

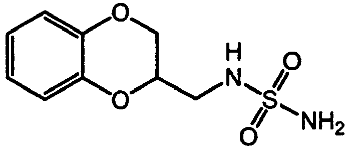

Racemic 2,3-dihydro-1,4-benzdioxin-2-ylmethylamine (4.4 g, 26 mmol) and sulfamide (5.1 g, 53 mmol) were combined in 1,4 dioxane (100 mL) and refluxed for 2 h. The reaction was cooled to room temperature and a small amount of solid was filtered and discarded. The filtrate was evaporated in vacuo and the residue was purified using flash column chromatography (DCM:Methanol - 10:1) to yield a white solid. The solid was recrystallized from DCM to yield the title compound as a white solid.
mp: 97.5 - 98.5°C
Elemental Analysis:
Anal Calc: C, 44.25; H, 4.95; N, 11.47; S, 13.13
Anal Found: C, 44.28; H, 4.66; N, 11.21; S, 13.15
H¹ NMR (DMSO d6) δ 6.85 (m, 4H), 6.68 (bd s, 3H, NH), 4.28 (m, 2H), 3.97 (dd, J = 6.9, 11.4 Hz, 1 H), 3.20 (m, 1 H), 3.10 (m, 1H).

### Example 3

### (Benzo[1,3]dioxol-2-ylmethyl)sulfamide (Compound #2)

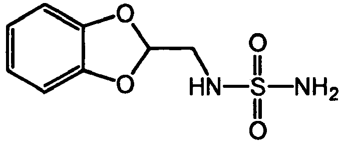

Catechol (10.26 g, 93.2 mmol), sodium methoxide (25% by weight in methanol, 40.3 g, 186 mmol), and methyl dichloroacetate (13.3 g, 93.2 mmol) were combined in dry methanol (100 mL). The solution was heated to reflux overnight. The reaction was cooled to room temperature, acidified by addition of concentrated hydrochloric acid and then reduced in volume under vacuum to about 50 mL. Water was added and the mixture was extracted with diethyl ether (3 x 100 mL). The combined organic solution was dried with MgSO₄, concentrated to a brown solid, and chromatographed (2% ethyl acetate in hexane) to yield benzo[1,3]dioxole-2-carboxylic acid methyl ester as a colorless oil.
MS (ESI): 195.10 (M+H⁺).
¹H NMR (300 MHz, CDCl₃), δ: 6.89 (broad, 4H), 6.29 (s, 1 H), 4.34 (q, *J* =7 Hz, 2H), 1.33 (t, *J* =7 Hz, 3H).

To benzo[1,3]dioxole-2-carboxylic acid methyl ester (7.21 g, 40.0 mmol) was added ammonium hydroxide (29% in water, 10 mL) and enough acetonitrile to make the mixture homogeneous (-5 mL). The solution was stirred for two hours at room temperature and then distilled water was added. Benzo[1,3]dioxole-2-carboxylic acid amide precipitated as a white solid and was collected by filtration and used without further purification.
MS (ESI): 160.00 (M+H⁺)
¹H NMR (300 MHz, DMSO), δ: 7.99 (s, broad, 1H), 7.72 (s, broad, 1H), 6.94 (m, 2H) 6.86 (m, 2H), 6.30 (s, 1H).

Benzo[1,3]dioxole-2-carboxylic acid amide (5.44 g, 32.9 mmol) was dissolved in tetrahydrofuran (THF, 100 mL). Lithium aluminum hydride (LAH, 1 M in THF, 39.5 mL, 39.5 mmol) was added slowly to the solution at room temperature. The reaction was stirred at room temperature for 24 hours. Distilled water was added to destroy the excess LAH. Aqueous sodium hydroxide (3.0 M, 100 mL) was added and the solution was extracted with ethyl acetate (3 x 100 mL). The combined organic solution was washed with water and dried over MgSO₄. The solvent was evaporated to yield C-benzo[1,3]dioxol-2-yl-methylamine as a colorless oil.
MS (ESI): 152.1 (M+H⁺)
¹H NMR (300 MHz, CDCl₃), δ: 6.87 (m, 4H), 6.09 (t, *J* = 4 Hz, 1H), 3.13 (d, *J* = 4 Hz, 2H)

C-Benzo[1,3]dioxol-2-yl-methylamine (2.94 g, 19.4 mmol) and sulfamide (3.74 g, 38.9 mmol) were combined in dry dioxane (50 mL) and the solution was heated to reflux overnight. The reaction was concentrated and the residue was chromatographed (2% to 10% acetone in dichloromethane) to yield the title compound as a white solid.
MS (ESI): 230.0 (M+H⁺)
¹H NMR (300 MHz, CDCl₃), δ: 6.87 (m, 4H), 6.25 (t, *J* = 4 Hz, 1H), 4.79 (broad, 1 H), 4.62 (broad, 1 H), 3.64 (d, *J* = 4 Hz, 2H).

### Example 4

### (2S)-(-)-N-(2,3-Dihydro-benzo[1,4]dioxin-2-ylmethyl)-sulfamide (Compound #4)

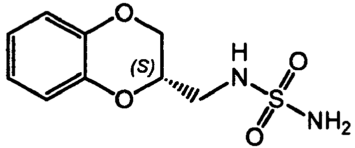

Catechol (13.2 g, 0.12 mol) and potassium carbonate (16.6 g, 0.12 mol) were stirred in DMF (250 mL) and (2R)-glycidyl tosylate (22.8 g, 0.10 mol) was added and the reaction was stirred at 60°C for 24 h. The reaction was cooled to room temperature and diluted with ice water (1 L) and extracted with diethyl ether (4 times). The combined organic solution was washed 3 times with 10% potassium carbonate, once with water, once with brine and evaporated in vacuo to yield a white solid which was purified by flash column chromatography (DCM:Methanol - 50:1) to yield ((2S)-2,3-dihydro-benzo[1,4]dioxin-2-yl)-methanol as a solid.

The solid (13.3 g, 68 mmol) was dissolved in pyridine (85 mL) cooled to 0°C, p-toluenesulfonyl chloride (13.0 g, 68 mmol) was added and the reaction mixture stirred at room temperature for 20h. The reaction was diluted with diethyl ether (1 L) and 1 N HCl (1.2 L). The organic layer was separated and washed 2 times with 1 N HCl (500 mL), 4 times with water (150 mL), once with brine, dried (MgSO₄) and evaporated in vacuo to yield a white solid which was purified by flash column chromatography (Hept:EA - 2:1) to yield toluene-4-sulfonic acid (2S)-2,3-dihydro-benzo[1,4]dioxin-2-ylmethyl ester as a white solid.

The white solid was combined with potassium phthalimide (14.4 g, 78 mmol) in DMF (250 mL) and heated to reflux for 1 h, cooled to room temperature and poured into vigorously stirring water (1.5 L) and stirred 30 min. White solid was filtered and the solid was washed several times with water, 2% NaOH, and water again and let air dry to yield a (2S)-2-(2,3-Dihydro-benzo[1,4]dioxin-2-ylmethyl)-isoindole-1,3-dione as white powdery solid.

The powdery white solid was combined with hydrazine (2.75 g, 86 mmol) in EtOH (225 mL) and heated at reflux for 2 h, cooled to room temperature and 1 N HCl added to pH 1.0 and stirred for 15 min. White solid was filtered and washed with fresh EtOH (solid discarded) and the filtrate was evaporated in vacuo to a solid, which was partitioned between diethyl ether and dilute aqueous NaOH. The diethyl ether solution was dried (Na₂SO₄) and evaporated in vacuo to a yield a light yellow oil. The oil was purified by flash column chromatography (DCM:MeOH - 10:1) to yield an oil. A portion of the oil (4.82 g, 29 mmol) in 2-propanol (250 mL) was treated with 1 N HCl (30 mL) and heated on steambath until homogeneous and then let cool to room temperature. After 3 h, the mixture was ice cooled for 2 h. A white flaky solid (the corresponding HCl salt of (2S)-C-(2,3-Dihydro-benzo[1,4]dioxin-2-yl)-methylamine) was filtered off and then recrystallized again from 2-propanol to yield a white solid.
[α]_{D} = -69.6 (c = 1.06, EtOH)

The white solid was partitioned between DCM and dilute NaOH, and the DCM was dried (NaSO₄) and evaporated in vacuo to yield (2S)-C-(2,3-Dihydro-benzo[1,4]dioxin-2-yl)-methylamine as an oil.

### [α]_{D} = -57.8 (c = 1.40, CHCl₃)

The oil (2.1 g, 12.7 mmol) and sulfamide (2.44 g, 25.4 mmol) were refluxed in dioxane (75 mL) for 2 h and the crude product was purified by flash column chromatography (DCM:MeOH 10:1) to yield a white solid, which was recrystallized from DCM to yield the title compound as a white crystalline solid.
mp 102-103°C
[α]_{D} = -45.1° (c = 1.05, M);
¹H NMR (DMSOd6) δ 6.86 (m, 4H), 6.81 (bd s, 3H, NH), 4.3 (m, 2H), 3.97 (dd, J = 6.9, 11.4 Hz, 1H), 3.20 (dd, J = 5.5, 13.7 Hz, 1H), 3.10 (dd, J = 6.9, 13.7Hz, 1H)
Elemental Analysis:
Anal Calc: C, 44.25; H, 4.95; N, 11.47; S, 13.13
Anal Found: C, 44.20; H, 4.69; N, 11.40; S, 13.22.

### Example 5

### N-(2,3-Dihydro-benzo[1,4]dioxin-2-ylmethyl)-N',N' dimethylsulfamide (Compound #6)

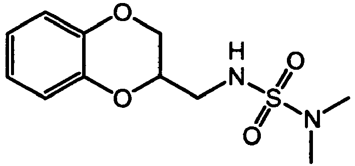

Racemic 2,3-dihydro-1,4-benzdioxin-2-ylmethylamine (8.25 g, 5.0 mmol) and triethylamine (1.52 g, 15 mmol) were combined in DMF (10 mL) and cooled in an ice bath as dimethylsulfamoyl chloride (1.44 g, 10 mmol) was added. The reaction mixture was then stirred for 3 hr with continued cooling. The reaction mixture was partitioned between ethyl acetate and water, and the ethyl acetate solution was washed with brine, dried (MgSO₄) and evaporated in vacuo to yield an oil. The oil was purified using flash column chromatography (methyl acetate:Heptane - 1:1) to yield a white solid, which was recrystallized (ethyl acetate/Hexane) to yield the title compound as a white floccular solid.
mp 76 - 78°C
MS 273 (MH⁺)
Elemental Analysis:
Anal Calc: C, 48.52; H, 5.92; N, 10.29; S, 11.78
Anal Found: C, 48.63; H, 5.62; N, 10.20; S, 11.90
¹H NMR (CDCl₃) δ 6.87 (m, 4H), 4.59 (bd m, 1H, NH), 4.35 (m, 1H), 4.27 (dd, J = 2.3, 11.4 Hz, 1 H), 4.04 (dd, J = 7.0, 11.4, 1H), 3.36 (m, 2H), 2.82 (s, 6H).

### Example 6

### N-(2,3-Dihydro-benzo[1,4]dioxin-2-ylmethyl)-N-methylsulfamide (Compound #7)

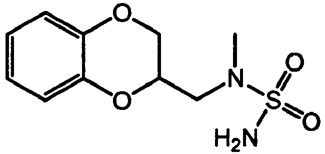

Racemic 2,3-dihydro-1,4-benzdioxin-2-ylmethylamine (825 mg, 5 mmol) was dissolved in ethyl formate (15 mL), refluxed for 30 min and evaporated in vacuo to yield N-(2,3-dihydro-benzo[1,4]dioxin-2-ylmethyl)-formamide as an oil.

The oil in diethyl ether (25 mL) was treated with 1 M LAH in THF (9.0 mL, 9.0 mmol) at 0°C and stirred for 5 h at room temperature. The reaction was cooled in an ice bath and quenched with water (0.50 mL), followed by 3 N NaOH (0.50 mL) and water (0.50 mL). The mixture was then stirred at room temperature for 1 h. Solid was filtered and the filtrate was evaporated in vacuo to yield a residue which was partitioned between 1 N HCl and diethyl ether. The aqueous phase was basified with 1 N NaOH and extracted with diethyl ether. The organic phase was dried (MgSO₄) and evaporated in vacuo to yield (2,3-dihydro-benzo[1,4]dioxin-2-ylmethyl)-methyl-amine as an oil.
MS 180 (MH⁺)
¹H NMR (CDCl₃) δ 6.85 (m, 4H), 4.30 (m, 2H), 4.02 (dd, J = 7.9, 11.6 Hz, 1 H), 2.85 (m, 2H), 2.50 (s, 3H)

The oil (380 mg, 2.1 mmol) and sulfamide (820 mg, 8.5 mmol) were combined in dioxane (15 mL), refluxed for 1.5 h and evaporated in vacuo to yield a crude residue. The residue was purified via column chromatography (ethyl acetate/Heptane 1:1) and the resultant solid was recrystallized from ethyl acetate/Hexane to yield the title compound as a white solid.
mp 97-98°C
MS 257 (M⁻¹)
Elemental Analysis:
Anal Calc: C, 46.50; H, 5.46; N, 10.85; S, 12.41
Anal Found: C, 46.48; H, 5.65; N, 10.90; S, 12.07
¹H NMR (CDCl₃) δ 6.86 (m, 4H), 4.52 (bs, 2H), 4.46 (m, 1 H), 4.29 (dd, J = 2.3, 11.5 Hz, 1 H), 4.05 (dd, J = 6.5, 11.5 Hz, 1 H), 3.51 (dd, J = 6.7, 14.9 Hz, 1 H), 3.40 (dd, J = 5.9, 14.9 Hz, 1 H), 2.99 (s, 3H).

### Example 7

### (2S)-(-)-N-(6-Chloro-2,3-dihydro-benzo[1,4]dioxin-2-ylmethyl)-sulfamide (Compound #8)

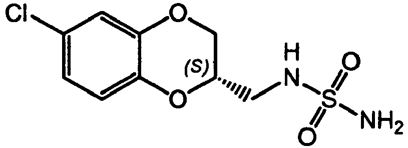

Following the procedure outlined in Example 4 above, 4-chlorocatechol was reacted to yield a mixture of (2S)-C-(7-Chloro-2,3-dihydro-benzo[1,4]dioxin-2-yl)-methylamine and (2S)-C-(6-Chloro-2,3-dihydro-benzo[1,4]dioxin-2-yl)-methylamine (ca. 3:1 ratio of 6-chloro:7-chloro isomers by RP HPLC).

The mixture was dissolved in 2-propanol (100 mL) and 1 N HCl in diethyl ether was added until pH = 1.0 was attained. The hydrochloride salt that precipitated was filtered (2.65 g) and re-crystallized from methanol/IPA to yield white crystals. The white crystals were partitioned between DCM and dilute NaOH. The DCM was dried and evaporated in vacuo to yield purified (2S)-C-(6-Chloro-2,3-dihydro-benzo[1,4]dioxin-2-yl)-methylamine as an oil.
[α]_{D} = -67.8 (c = 1.51, CHCl₃)

The oil (7.75 mmol) and sulfamide (1.50 g, 15.5 mmol) were combined in dioxane (50 mL) and refluxed for 2.0 h, cooled to room temperature and evaporated in vacuo to yield a solid. The product was purified via flash column using DCM/methanol 20:1 to yield the title compound as a white solid.
MS 277 (M⁻¹)
[α]_{D} = -59.9° (c = 1.11, M)
¹H NMR (CDCl₃) δ 6.90 (d, J = 2.2 Hz, 1 H), 6.81 (m, 2H), 4.76 (m, 1 H), 4.55 (s, 2H), 4.40 (m, 1H), 4.29 (dd, J = 2.4, 11.5 Hz, 1 H), 4.05 (dd, J = 7.1, 11.5 Hz, 1 H), 3.45 (m, 2H)
Elemental Analysis:
Anal Calc: C, 38.78; H, 3.98; N, 10.05
Anal Found: C, 38.80; H, 3.67; N, 9.99.

The filtrates of the crystallized hydrochloride salt of (2S)-C-(6-Chloro-2,3-dihydro-benzo[1,4]dioxin-2-yl)-methylamine prepared above were recovered (ca. 1:1 of 6-chloro:7-chloro isomers) and evaporated in vacuo to yield a solid, which was partitioned between DCM (200 mL) and dilute NaOH (0.5 M, 50 mL). The DCM solution was washed once with brine, dried (Na₂SO₄) and evaporated in vacuo to yield an oil, which was purified via reverse phase HPLC (10 - 50% ACN with 0.16% TFA in water with 0.20% TFA) to yield (2S)-C-(7-Chloro-2,3-dihydro-benzo[1,4]dioxin-2-yl)-methylamine as a residue.

The residue was combined with sulfamide (0.90 g, 9.4 mmol) in dioxane (25 mL) and refluxed for 2.5 h, cooled to room temperature and evaporated in vacuo to yield an oil. The oil was purified by flash column chromatography using DCM/methanol - 10:1 to yield (2S)-(-)-N-(7-Chloro-2,3-dihydro-benzo[1,4]dioxin-2-ylmethyl)-sulfamide as a white solid.
MS 277 (M⁻¹)
¹H NMR (CDCl₃/CD₃OD) δ 6.88 (d, J = 0.7 Hz, 1H), 6.81 (m, 2H), 4.37 (m, 1 H), 4.30 (dd, J = 2.3, 11.6 Hz, 1 H), 4.04 (dd, J = 7.0, 11.6 Hz, 1 H), 3.38 (m, 2H).

### Example 8

### Chroman-2-ylmethylsulfamide (Compound #10)

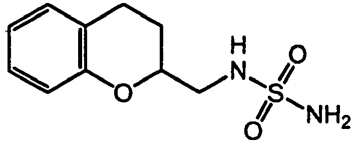

Chroman-2-carboxylic acid (4.5 g, 25 mmol) and HOBT (3.86 g, 25 mmol) were combined in DCM (40 mL) and DMF (10 mL). Dimethylaminopropyl ethylcarbodiimide (EDC, 4.84 g, 25 mmol) was added at room temperature and the reaction mixture was stirred for 30 min. Ammonium hydroxide (2.26 mL, 33.4 mmol) was added and the reaction mixture was stirred for 16h. The reaction mixture was diluted with DCM (50 mL) and water (50 mL) and the pH of the mixture was adjusted to about pH = 3.0 with 1 N HCl. The DCM was separated and the aqueous phase extracted twice with DCM. The combined DCM phase was dried (Na₂SO₄) and evaporated in vacuo to yield an oil, which was purified with flash column chromatography (ethyl acetate) to yield an oil.

The oil (5.35 g, 30 mmol) in THF (90 mL) was stirred as 1 M LAH in THF (36 mL, 36 mmol) was added and the reaction mixture was then stirred at room temperature for 20 h. The reaction was quenched with water, stirred for 2 hours, the solution decanted, dried (Na₂SO₄) and evaporated in vacuo to yield C-chroman-2-yl-methylamine as an oily amine.

The oily amine (1.63 g, 10 mmol) and sulfamide (1.92 g, 20 mmol) were combined in dioxane (50 mL) and brought to reflux for 2 h. The solution was cooled and evaporated in vacuo to yield an oil, which was purified via column chromatography (DCM:Methanol 10:1) to yield a white solid. The solid was recrystallized from ethyl acetate/hexane to yield chroman-2-ylmethylsulfamide as a white solid.
mp 100-101 °C
MS 241 (M⁻¹)
Elemental Analysis:
Anal Calc: C, 49.57; H, 5.82; N, 11.56; S, 13.23
Anal Found: C, 49.57; H, 5.80; N, 11.75; S, 13.33.

### Example 9

### 2-(2,3-Dihydro-benzo[1,4]dioxin-2-yl)-ethylsulfamide (Compound #16)

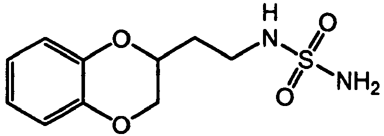

Potassium cyanide (2.05 g, 31.5 mmol) was added to 2-bromomethyl-(2,3 dihydrobenzo[1,4]dioxine) (6.87 g, 30 mmol) in DMSO (90 mL) and stirred at ambient temperature for 20 h. The reaction mixture was then diluted with water (250 mL) and extracted twice with diethyl ether. The diethyl ether was washed with water, then washed twice with brine, dried (Na₂SO₄) and evaporated in vacuo to yield 2-cyanomethyl-(2,3 dihydrobenzo[1,4]dioxine) as a white solid.
¹H NMR (CDCl₃) δ 6.89 (m, 4H), 4.50 (m, 1 H), 4.31 (dd, J = 2.3, 11.5 Hz, 1 H), 4.08 (dd, J = 6.2, 11.6 Hz, 1 H), 2.78 (d, J = 6.1, Hz, 2H)

The 2-cyanomethyl-(2,3 dihydrobenzo[1,4]dioxine) was dissolved in THF (50 mL) and 1 M BH₃ in THF (80 mL, 80 mmol) was added and the reaction mixture refluxed for 5 h, then stirred at ambient temperature for 16h. With ice bath cooling, 2N HCl was added until pH = 1.0 was achieved. The reaction mixture was then stirred for 1 h at room temperature and evaporated in vacuo to yield an oil. The oil was partitioned between 3N NaOH and diethyl ether, and the diethyl ether solution was washed with brine, dried (Na₂SO₄) and evaporated in vacuo to yield crude 2-(2,3 dihydrobenzo[1,4]dioxin-2-yl)ethylamine.
MS (M+H)⁺ 180.

The crude 2-(2,3 dihydrobenzo[1,4]dioxin-2-yl)ethylamine in dioxane (100 mL) was combined with sulfamide (3.0 g, 31 mmol) and heated to reflux for 2 h. The solution was cooled and evaporated in vacuo to yield an orange solid, which was purified by column chromatography (DCM:MeOH - 10:1) to yield a white solid. The solid was re-crystallized from DCM to yield the title compound as a solid.
MS (M-1) 257
MP 101 - 103°C (corr)
¹H NMR (CDCl₃): δ 6.86 (m, 4H), 4.70 (m, 1H), 4.52 (s, 2H), 4.30 (m, 2H), 3.94 (dd, J = 7.4, 11.3 Hz, 1 H), 3.43 (dd, J = 6.4, 12.9 Hz, 2H), 1.94 (dd, J = 6.5, 12.9, 2H).
Elemental Analysis:
Measured: C, 46.48; H, 5.60; N, 10.81; S, 12.41
Calculated: C, 46.50; H, 5.46; N, 10.85; S, 12.41

### Example 10

### (2S)-(-)-N-(6,7 Dichloro-2,3-dihvdro-benzo[1,4]dioxin-2-ylmethyl)-sulfamide (Compound #29)

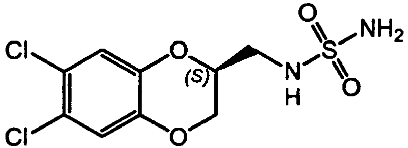

4,5 Dichloroatechol (8.6 g, 48 mmol) and potassium carbonate (6.64 g, 48 mmol) were stirred in DMF (200 mL). (2R)-Glycidyl tosylate (9.12 g, 40 mmol) was added and the reaction mixture was stirred at 60°C for 24 h. The reaction mixture was cooled to room temperature and then diluted with ice water (600 mL) and extracted with diethyl ether (4 times). The combined organic solution was washed 3 times with 10% potassium carbonate, twice with brine, dried (MgSO₄) and evaporated in vacuo to yield a viscous oil of (2S)-2-(6,7-dichloro-2,3-dihydro-benzo[1,4]dioxine) methanol.

The (2S)-2-(6,7 dichloro-2,3-dihydro-benzo[1,4]dioxine) methanol oil (6.4 g, 27 mmol) was dissolved in pyridine (50 mL) cooled to 0°C. Then, p-toluenesulfonyl chloride (5.2 g, 27 mmol) was added and the reaction mixture was stirred at room temperature for 20h. The reaction mixture was diluted with diethyl ether and 1 N HCl (750 mL) and the organic layer was separated and washed 2 times with 1 N HCl (250 mL), once with water (150 mL), twice with brine, dried (MgSO₄) and evaporated in vacuo to yield light yellow solid of toluene-4-sulfonic acid (2S)-6,7-dichloro-2,3-dihydro-benzo[1,4]dioxin-2-ylmethyl ester.
¹H NMR (CDCl3): δ 7.79 (d, J = 8.3 Hz, 2H), 7.36 (d, J = 8.0 Hz, 2H), 6.94 (s, 1 H), 6.83 (s, 1 H), 4.37 (m, 1 H), 4.2 (m, 3H), 4.03 (dd, J = 6.3, 11.7 Hz, 1 H), 2.47 (s, 3H).

Toluene-4-sulfonic acid (2S)-6,7-dichloro-2,3-dihydro-benzo[1,4]dioxin-2-ylmethyl ester (8.0 g, 20.5 mmol) was combined with potassium phthalimide (6.1 g, 33 mmol) in DMF (75 mL) and heated to reflux for 1 h, cooled to room temperature and poured into vigorously stirring water (0.5 L) and then stirred 30 min. White solid was filtered and the solid was washed several times with water, 2% NaOH, and water again and then let air dry to yield (2S)-2-(6,7-dichloro-2,3-dihydro-benzo[1,4]dioxin-2-ylmethyl)-isoindole-1,3-dione (6.0 g, 80%) as a white powdery solid.

The white powdery solid was combined with hydrazine (1.06 g, 33 mmol) in EtOH (80 mL) and heated at reflux for 2 h, then cooled to room temperature. 1 N HCl was added to adjust the reaction mixture's pH to pH 1.0 and the reaction mixture was then stirred for 15 min. White solid was filtered and washed with fresh EtOH (solid discarded) and the filtrate was evaporated in vacuo to a solid, which was partitioned between diethyl ether and dilute aqueous NaOH. The diethyl ether solution was dried (Na₂SO₄) and evaporated in vacuo to a yield a viscous oil of (2S)-2-aminomethyl-(6,7-dichloro-2,3-dihydro-benzo[1,4]dioxine).
¹H NMR (CDCl3): δ 6.98 (s, 1H), 6.96 (s, 1H), 4.25 (dd, J = 2.0, 11.2 Hz, 1 H), 4.15 (m, 1H), 4.0 (m, 1 H), 2.97 (d, J = 5.5 Hz, 2H)

A portion of the oil (3.8 g, 16 mmol) and sulfamide (3.1 g, 32.4 mmol) were refluxed in dioxane (100 mL) for 2 h and the crude product was purified by flash column chromatography (DCM:MeOH 20:1) to yield the title compound as a white solid, which was recrystallized from ethyl acetate / hexane to yield the title compound as a white crystalline solid.
MS [M-H]⁻ 311.0
mp 119-121 °C
[α]_{D} = -53.4° (c = 1.17, M)
¹H NMR (DMSOd6): δ 7.22 (s, 1 H), 7.20 (s, 1H), 6.91 (bd s, 1H), 6.68 (bd s, 2H), 4.35 (m, 2H), 4.05 (dd, J = 6.5, 11.5 Hz, 1H), 3.15 (m, 2H) Elemental Analysis:
Elemental Analysis:
Measured: C, 34.52; H, 3.22; N, 8.95; Cl, 22.64; S, 10.24
Calculated: C, 34.64; H, 2.68; N, 8.87; Cl, 22.94; S, 10.35.

### Example 11

### (2S)-(-)-N-(7-Amino-2,3-dihydro-benzo[1,4]dioxin-2-ylmethyl)-sulfamide (Compound #36)

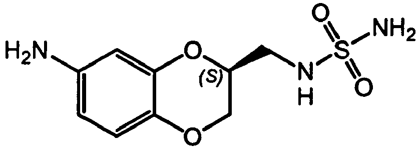

(2S)-(-)-N-(2,3-Dihydro-7-nitro-benzo[1,4]dioxin-2-ylmethyl)-sulfamide (1.2 g, 4.15 mmol), was prepared from 4-nitrocatechol according to the process outlined in Example 4. The (2S)-(-)-N-(2,3-Dihydro-7-nitro-benzo[1,4]dioxin-2-ylmethyl)-sulfamide, was then combined with 10% Pd/C in methanol (120 mL) and shaken under hydrogen atmosphere (39 psi) at room temperature for 3 h. The solids were filtered and washed with 10% M in DCM and the filtrate was evaporated in vacuo to yield crude product. The crude product was dissolved in 0.2 N HCl (25 mL), frozen and lyophilized to yield the title compound as a white flaky solid, as the corresponding hydrochloride salt.
MS (M+H)⁺ 260
¹H NMR (DMSO d6): δ 10.2 (bd s, 3H), 6.86 (m, 1H), 6.85 (s, 1H), 6.74 (dd, J = 2.5, 8.4 Hz, 1 H), 4.22 (m, 2H), 3.88 (dd, J = 6.7, 11.4 Hz, 1 H), 3.04 (m, 2H)

### Example 12

### (2S)-(-)-N-(7-Methyl-2,3-dihydro-benzo[1,4]dioxin-2-ylmethyl)-sulfamide (Compound #19)

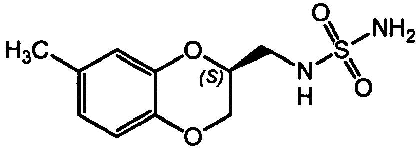

Title compound was prepared according to the procedure described in Example 4 above, starting with 4-methylcatechol, to yield a white solid, which was recrystallized from ethyl acetate/ hexane to yield the title compound as a white solid.
MS [M-H]⁻ 257
¹H NMR (CDCl3): δ 6.76 (m, 1 H), 6.66 (m, 2H), 4.80 (m, 1H), 4.57 (bd s, 1H), 4.40 (m, 1H), 4.28 (m, 1 H), 4.03 (dd, J = 6.9, 11.4 Hz, 1 H), 3.45 (m, 2H), 2.25 (s, 3H).
Elemental Analysis
Calculated: C, 46.50; H, 5.46; N, 10.85; S, 12.41
Found: C, 46.65; H, 5.60; N, 10.84; S, 12.61.

### Example 13

### Cortical Spreading Assay as Model of Migraine

Cortical spreading depression (CSD) has been implicated in migraine and as a headache trigger, and can be evoked in experimental animals by electrical or chemical stimulation (Kunkler & Kraig, 2003; Lauritzen et al., 1982; Moskowitz, 1984). Moreover, migraine prophylactic drugs have been shown to elevate CSD threshold thereby decreasing the number of CSD's, which is considered a potential mechanism by which they reduce the frequency of migraine attacks (Ayata et al., Ann Neurol in press).

Adult male Sprague-Dawley rats (250-600g) were divided into two treatment groups: vehicle (0.5% methylcellulose; n=13) and Compound #8 (100 mg/kg/day, p.o.; n=7). Results were compared to historical positive controls with valproic acid at 200 mg/kg/day, i.p.

Vehicle or Compound #8 was administered orally once a day for 35 days. On the last day of treatment, rats continued to receive food and water ad libitum, and were given their daily oral dose of vehicle or Compound #8 approximately 1.5h prior to CSD testing. Rats were anesthetized using isoflurane and intubated via a tracheotomy for mechanical ventilation. Body temperature, blood pressure and heart rate were monitored throughout the procedure to ensure homeostasis. Rats were placed in a stereotaxic frame and three burr holes were drilled under saline cooling over the right hemisphere at the following coordinates (mm from bregma): (1) posterior 4.5, lateral 2.0 (occipital cortex): KCL application; (2) posterior 0.5, lateral 2.0 (parietal cortex): recording site 1; (3) anterior 2, lateral 2 (frontal cortex): recording site 2. Dura overlying the occipital cortex was gently removed. The steady (DC) potential and electrocorticogram (EcoG) were recorded with glass micropipettes filled with 200mM NaCl, 300uM below the dural surface. An Ag/AgCl reference electrode was placed subcutaneously in the neck. Following surgical preparation, the cortex was allowed to recover for 30 minutes under saline irrigation. Cortical spreading depressions were initiated by placing a cotton ball soaked with 1 M KCI on the pial surface. The number of KCL-induced CSDs was counted for 2 hours. Propagation speed was calculated from the distance (mm) between the recording electrodes 1 and 2, divided by the latency (min) between the CSDs recorded at these sites.

As shown in Table 3 below, the number of cortical spreading depressions evoked by topical KCI application was 16 ± 3 and 14 ± 3 for the vehicle and Compound #8 treated animals, respectively. Although there appeared to be a difference between the vehicle group and the test compound, these results did not reach statistical significance (p=0.12, Kruskal-Wallis One Way Analysis of Variance on Ranks). There was also a trend in reduction of propagation speed from approximately 3.2 ± 1 mm/min (vehicle) to 2.7 ± 0.4 mm/min (Compound #8) (p=0.06, Kruskal- Wallis One Way Analysis of Variance). Physiological monitoring, including arterial pH, pCO₂, pO₂ and blood pressure did not differ between the groups.

As a positive control, valproic acid (200 mg/kg/day, i.p.) was administered as previously reported (Ayata et al., Ann Neurol, in press) for a period of 4 weeks. Saline was used as vehicle control. The number of cortical spreading depressions in the saline and valproate groups were 18 and 14, respectively (p<0.05, Mann-Whitney Rank Sum Test).

Based on these results, the inventors believe that Compound #8 would become more effective after a longer period of treatment or at a higher dose, especially in view of the trend in reduction of propagation speed.

**Table 4:**

| **Effect of Compound #1 on KCl-Induced Cortical Spreading Depression** | | | | | | |
|---|---|---|---|---|---|---|
| **Treatment** | **Body weight (g)** | | **CSDs (2h) ≥5mV** | | **Propagation Speed (mm/min)** | |
| Vehicle (0.5% MC) | 401 ± 40 | | 16±3 | | 3.2 ± 1 | |
| Compound #8 | 418 ± 31 | | 14 ± 3 | | 2.7 ± 0.4 | |

| | **Systemic Physiology** | | | | | |
|---|---|---|---|---|---|---|
| | **pH** | **pCO₂** | | **PO₂** | | **BP** |
| Vehicle (0.5% MC) | 7.44 ± 0 | 38 ± 3 | | 162±9 | | 105 ± 14 |
| Compound #8 | 7.42 ± 0.03 | 39 ± 3 | | 157 ± 18 | | 107 ± 18 |

### Example 14

As a specific embodiment of an oral composition, 100 mg of the Compound #8 prepared as in Example 7 is formulated with sufficient finely divided lactose to provide a total amount of 580 to 590 mg to fill a size O hard gel capsule.

## Claims

1. A compound of the formula (I) or a pharmaceutically acceptable salt thereof for use in a method of treating migraine 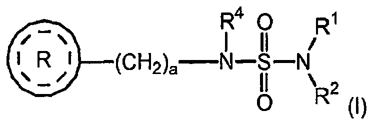 wherein
R¹ and R² are each independently selected from the group consisting of hydrogen and lower alkyl;
R⁴ is selected from the group consisting of hydrogen and lower alkyl; a is an integer from 1 to 2; 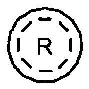 is selected from the group consisting of 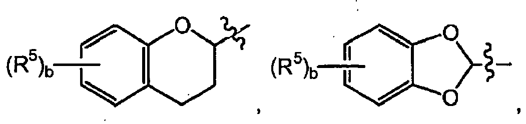 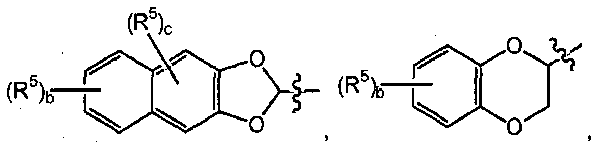 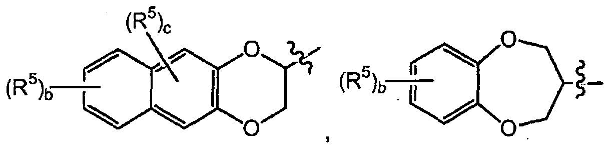 and 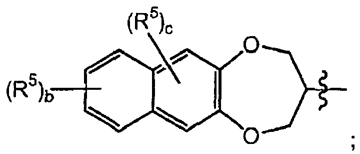 wherein b is an integer from 0 to 4; and wherein c is an integer from 0 to 2;
each R⁵ is independently selected from the group consisting of halogen, lower alkyl and nitro;
provided that when 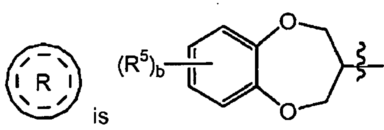 or 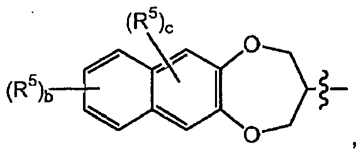 then a is 1: wherein lower alkyl means a carbon chain composition of 1-4 carbon atoms.

2. The compound or pharmaceutically acceptable salt thereof for use as in Claim 1, wherein
R¹ and R² are each independently selected from the group consisting of hydrogen and lower alkyl;
R⁴ is selected from the group consisting of hydrogen and lower alkyl; a is an integer from 1 to 2; 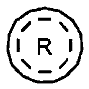 is selected from the group consisting of 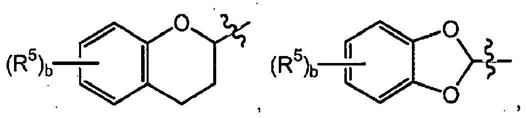 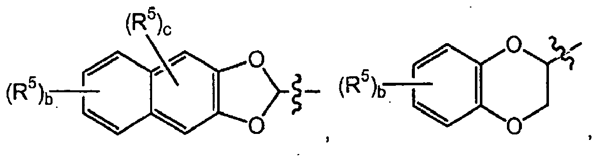 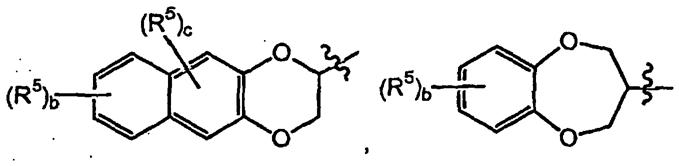 and 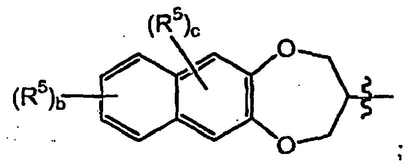 wherein b is an integer from 0 to 2; and wherein c is an integer from 0 to 1;
each R⁵ is independently selected from the group consisting of halogen, lower alkyl and nitro;
provided that when 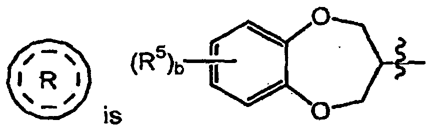 or 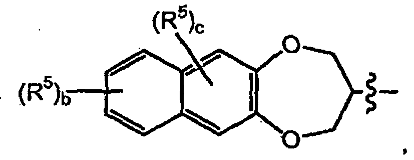 then a is 1; wherein lower alkyl means a carbon chain composition of 1-4 carbon atoms.

3. The compound or pharmaceutically acceptable salt thereof for use as in Claim 2, wherein
R¹ and R² are each independently selected from the group consisting of hydrogen and lower alkyl;
R⁴ is selected from the group consisting of hydrogen and lower alkyl;
a is an integer from 1 to 2; 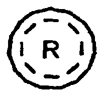 is selected from the group consisting of 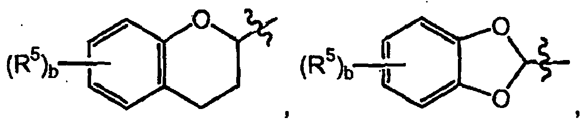 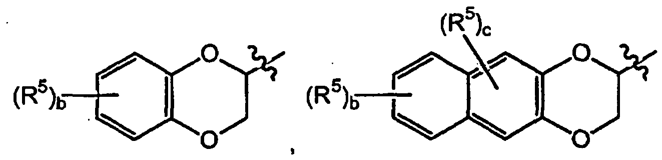 and 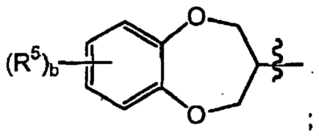 wherein b is an integer from 0 to 2; and wherein c is 0;
each R⁵ is independently selected from the group consisting of halogen, lower alkyl and nitro;
provided that when 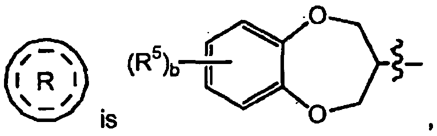 then a is 1; wherein lower alkyl means a carbon chain composition of 1-4 carbon atoms.

4. The compound or pharmaceutically acceptable salt thereof for use as in Claim 3, wherein
R¹ and R² are each independently selected from the group consisting of hydrogen and lower alkyl;
R⁴ is selected from the group consisting of hydrogen and methyl; a is an integer from 1 to 2; 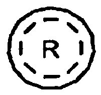 is selected from the group consisting of 2-(2,3-dihydro-benzo[1,4]dioxinyl), 2-(benzo[1,3]dioxolyl), 2-(3,4-dihydro-2H-benzo[1,4]dioxepinyl), 2-(2,3-dihydro-benzo[1,4]dioxinyl), 2-(6-chloro-2,3-dihydro-benzo[1,4]dioxinyl), 2-(6-fluoro-2,3-dihydro-benzo[1,4]dioxinyl), 2-(chromanyl), 2-(5-fluoro-2,3-dihydro-benzo[1,4]dioxinyl), 2-(7-chloro-2,3-dihydro-benzo[1,4]dioxinyl), 2-(6-chloro-benzo[1,3]dioxolyl), 2-(7-nitro-2,3-dihydro-benzo[1,4]dioxinyl), 2-(7-methyl-2,3-dihydro-benzo[1,4]dioxinyl), 2-(5-chloro-2,3-dihydro-benzo[1,4]dioxinyl), 2-(6-bromo-2,3-dihydro-benzo[1,4]dioxinyl), 2-(6,7-dichloro-2,3-dihydro-benzo[1,4]dioxinyl), 2-(8-chloro-2,3-dihydro-benzo[1,4]dioxinyl), 2-(2,3-dihydro-naphtho[2,3-b][1,4]dioxinyl) and 2-(4-methyl-benzo[1,3]dioxolyl);
provided that when 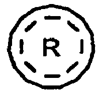 is 2-(3,4-dihydro-2H-benzo[1,4]dioxepinyl),
then a is 1; wherein lower alkyl means a carbon chain composition of 1-4 carbon atoms.

5. The compound or pharmaceutically acceptable salt thereof for use as in Claim 4, wherein
R¹ and R² are each independently selected from the group consisting of hydrogen and methyl;
R⁴ is selected from the group consisting of hydrogen and methyl;
a is an integer from 1 to 2; 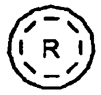 is selected from the group consisting of 2-(benzo[1,3]dioxolyl), 2-(2,3-dihydro-benzo[1,4]dioxinyl), 2-(2,3-dihydro-benzo[1,4]dioxinyl), 2-(6-chloro-2,3-dihydro-benzo[1,4]dioxinyl), 2-(7-chloro-2,3-dihydro-benzo[1,4]dioxinyl), 2-(7-methyl-2,3-dihydro-benzo[1,4]dioxinyl), 2-(6-bromo-2,3-dihydro-benzo[1,4]dioxinyl) and 2-(6,7-dichloro-2,3-dihydro-benzo[1,4]dioxinyl).

6. The compound or a pharmaceutically acceptable salt thereof for use as claimed in Claim 1, wherein the compound of formula (I) is selected from the group consisting of (2S)-(-)-N-(6-chloro-2,3-dihydro-benzo[1,4]dioxin-2-ylmethyl)-sulfamide; and pharmaceutically acceptable salts thereof.

7. A compound of the formula (II) or a pharmaceutically acceptable salt thereof for use in a method of treating migraine.

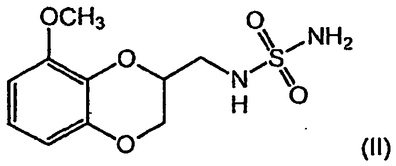

8. The compound for use as claimed in claim 6, wherein the compound of formula (I) is (2S)-(-)-N-(6-chloro-2,3-dihydro-benzo[1,4]dioxin-2-ylmethyl)-sulfamide:

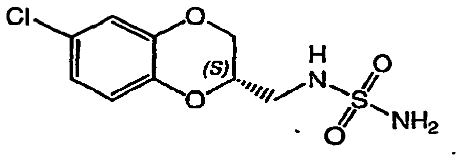

## Patentansprüche

1. Verbindung der Formel (I) oder ein pharmazeutisch unbedenkliches Salz davon zur Verwendung bei einer Methode zur Behandlung von Migräne: 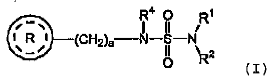 wobei
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht; 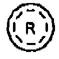 aus der Gruppe bestehend aus 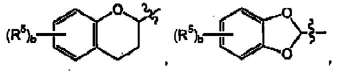 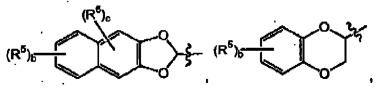 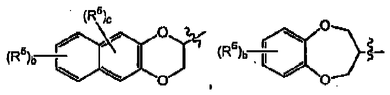 und 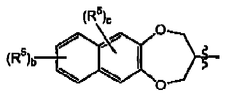 ausgewählt ist, wobei b für eine ganze Zahl von 0 bis 4 steht und c für eine ganze Zahl von 0 bis 2 steht;
R⁵ jeweils unabhängig voneinander aus der Gruppe bestehend aus Halogen, Niederalkyl und Nitro ausgewählt ist;
mit der Maßgabe, dass dann, wenn 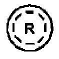 für 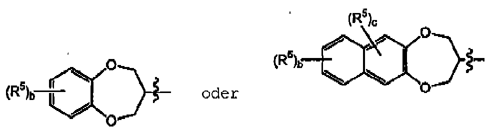 steht, a für 1 steht;
wobei Niederalkyl eine Kohlenstoffkettenzusammensetzung mit 1-4 Kohlenstoffatomen bedeutet.

2. Verbindung oder pharmazeutisch unbedenkliches Salz davon zur Verwendung nach Anspruch 1, wobei R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht; 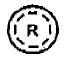 aus der Gruppe bestehend aus 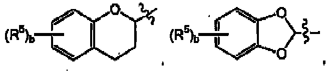 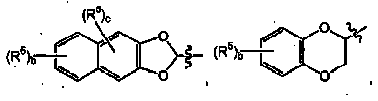 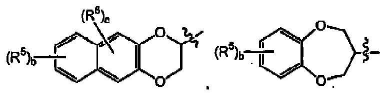 und 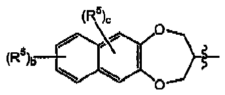 ausgewählt ist, wobei b für eine ganze Zahl von 0 bis 2 steht und c für eine ganze Zahl von 0 bis 1 steht;
R⁵ jeweils unabhängig voneinander aus der Gruppe bestehend aus Halogen, Niederalkyl und Nitro ausgewählt ist;
mit der Maßgabe, dass dann, wenn 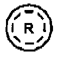 für 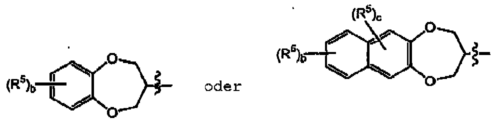 steht, a für 1 steht;
wobei Niederalkyl eine Kohlenskoffkettenzusammensetzung mit 1-4 Kohlenstoffatomen bedeutet.

3. Verbindung oder pharmazeutisch unbedenkliches Salz davon zur Verwendung nach Anspruch 2, wobei R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht; 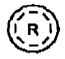 aus der Gruppe bestehend aus 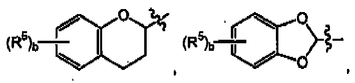 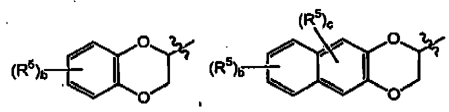 und 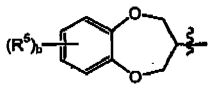 ausgewählt ist, wobei b für eine ganze Zahl von 0 bis 2 steht und c für 0 steht;
R⁵ jeweils unabhängig voneinander aus der Gruppe bestehend aus Halogen, Niederalkyl und Nitro ausgewählt ist;
mit der Maßgabe, dass dann, wenn 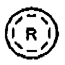 für 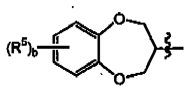 steht, a für 1 steht;
wobei Niederalkyl eine Kohlenstoffkettenzusammensetzung mit 1-4 Kohlenstoffatomen bedeutet.

4. Verbindung oder pharmazeutisch unbedenkliches Salz davon zur Verwendung nach Anspruch 3, wobei R¹ und R² jeweils unabhängig voneinander aus der Gruppe bestehend aus Wasserstoff und Niederalkyl ausgewählt sind;
R⁴ aus der Gruppe bestehend aus Wasserstoff und Methyl ausgewählt ist;
a für eine ganze Zahl von 1 bis 2 steht; 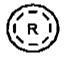 aus der Gruppe bestehend aus 2-(2,3-Dihydrobenzo[1,4]dioxinyl), 2-(Benzo[1,3]dioxolyl), 2-(3,4-Dihydro-2H-benzo[1,4]dioxepinyl), 2-(2,3-Dihydrobenzo[1,4]dioxinyl), 2-(6-Chlor-2,3-dihydrobenzo[1,4]dioxinyl), 2-(6-Fluor-2,3-dihydrobenzo[1,4]dioxinyl), 2-(Chromanyl), 2-(5-Fluor-2,3-dihydrobenzo[1,4]dioxinyl), 2-(7-Chlor-2,3-dihydrobenzo[1,4]dioxinyl), 2-(6-Chlorbenzo-[1,3]dioxolyl), 2-(7-Nitro-2,3-dihydrobenzo[1,4]-dioxinyl), 2-(7-Methyl-2,3-dihydrobenzo[1,4]-dioxinyl), 2-(5-Chlor-2,3-dihydrobenzo[1,4]-dioxinyl), 2-(6-Brom-2,3-dihydrobenzo[1,4]-dioxinyl), 2-(6,7-Dichlor-2,3-dihydrobenzo[1,4]-dioxinyl), 2-(8-Chlor-2,3-dihydrobenzo[1,4]-dioxinyl), 2-(2,3-Dihydronaphtho[2,3-b][1,4]-dioxinyl) und 2-(4-Methylbenzo[1,3]dioxolyl) ausgewählt ist; mit der Maßgabe, dass dann, wenn 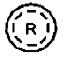 für 2-(3,4-Dihydro-2H-benzo[1,4]dioxinyl steht, a für 1 steht;
wobei Niederalkyl eine Kohlenstoffkettenzusammensetzung mit 1-4 Kohlenstoffatomen bedeutet.

5. Verbindung oder pharmazeutisch unbedenkliches Salz davon zur Verwendung nach Anspruch 4, wobei
R¹ und R² jeweils unabhängig voneinander aus der Gruppe bestehend aus Wasserstoff und Methyl ausgewählt sind;
R⁴ aus der Gruppe bestehend aus Wasserstoff und Methyl ausgewählt ist;
a für eine ganze Zahl von 1 bis 2 steht; 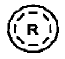 aus der Gruppe bestehend aus 2-(Benzo[1,3]-dioxolyl), 2-(2,3-Dihydrobenzo[1,4]dioxinyl), 2-(2,3-Dihydrobenzo[1,4]dioxinyl), 2-(6-Chlor-2,3-dihydrobenzo[1,4]dioxinyl), 2-(7-Chlor-2,3-dihydrobenzo[1,4]dioxinyl), 2-(7-Methyl-2,3-dihydrobenzo[1,4]dioxinyl), 2-(6-Brom-2,3-dihydro-benzo[1,4]dioxinyl) und 2-(6,7-Dichlor-2,3-dihydrobenzo[1,4]dioxinyl) ausgewählt ist.

6. Verbindung oder pharmazeutisch unbedenkliches Salz davon zur Verwendung nach Anspruch 1, wobei die Verbindung der Formel (I) aus der Gruppe bestehend aus (2S)-(-)-N-(6-Chlor-2,3-dihydrobenzo[1,4]-dioxin-2-ylmethyl)sulfamid und pharmazeutisch unbedenklichen Salzen davon ausgewählt ist.

7. Verbindung der Formel (II) oder pharmazeutisch unbedenkliches Salz davon zur Verwendung bei einer Methode zur Behandlung von Migräne:

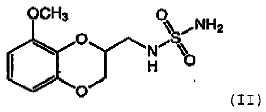

8. Verbindung zur Verwendung nach Anspruch 6, wobei es sich bei der Verbindung der Formel (I) um (2S)-(-)-N-(6-Chlor-2,3-dihydrobenzo[1,4]dioxin-2-ylmethyl)sulfamid handelt:

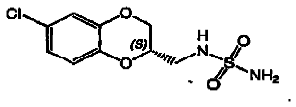

## Revendications

1. Composé représenté par la formule (I) 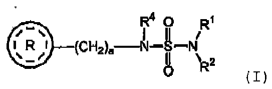 ou sel pharmaceutiquement acceptable de celui-ci destiné à être utilisé dans un procédé de traitement de la migraine
dans lequel
R¹ et R² sont chacun indépendamment choisis dans le groupe constitué par l'atome d'hydrogène et les groupes alkyle inférieurs ;
R⁴ est choisi dans le groupe constitué par l'atome d'hydrogène et les groupes alkyle inférieurs ;
a est un nombre entier de 1 à 2 ; 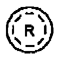 est choisi dans le groupe constitué par 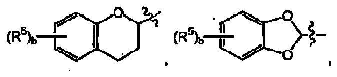 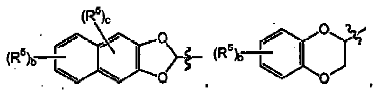 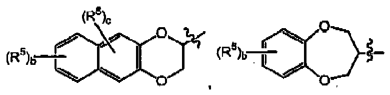 et 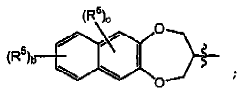 où
b est un nombre entier de 0 à 4 ;
c est un nombre entier de 0 à 2 ; et
chaque R⁵ est indépendamment choisi dans le groupe constitué par les atomes d'halogène, les groupes alkyle inférieurs et le groupe nitro ; à condition que lorsque 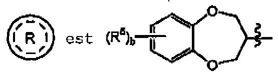 ou 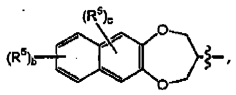 alors a vaille 1 ;
alkyle inférieur désignant une composition de chaîne carbonée constituée de 1-4 atomes de carbone.

2. Composé ou sel pharmaceutiquement acceptable de celui-ci destiné à être utilisé selon la revendication 1, dans lequel
R¹ et R² sont chacun indépendamment choisis dans le groupe constitué par l'atome d'hydrogène et les groupes alkyle inférieurs ;
R⁴ est choisi dans le groupe constitué par l'atome d'hydrogène et les groupes alkyle inférieurs ;
a est un nombre entier de 1 à 2 ; 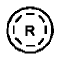 est choisi dans le groupe constitué par 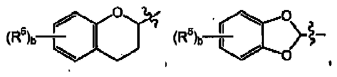 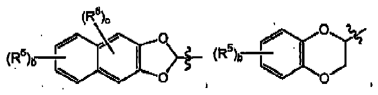 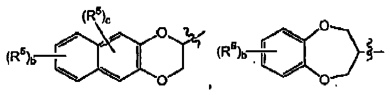 et 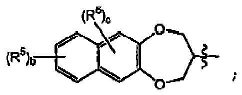 où
b est un nombre entier de 0 à 2 ;
c est un nombre entier de 0 à 1 ; et
chaque R⁵ est indépendamment choisi dans le groupe constitué par les atomes d'halogène, les groupes alkyle inférieurs et le groupe nitro ; à condition que lorsque 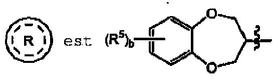 ou 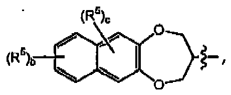 alors a vaille 1 ; alkyle inférieur désignant une composition de chaîne carbonée constituée de 1-4 atomes de carbone.

3. Composé ou sel pharmaceutiquement acceptable de celui-ci destiné à être utilisé selon la revendication 2, dans lequel
R¹ et R² sont chacun indépendamment choisis dans le groupe constitué par l'atome d'hydrogène et les groupes alkyle inférieurs ;
R⁴ est choisi dans le groupe constitué par l'atome d'hydrogène et les groupes alkyle inférieurs ;
a est un nombre entier de 1 à 2 ; 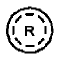 est choisi dans le groupe constitué par 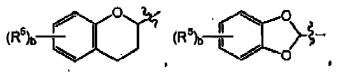 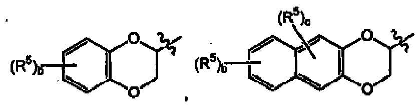 et 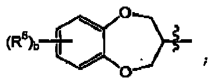 où
b est un nombre entier de 0 à 2 ;
c vaut 0 ; et
chaque R⁵ est indépendamment choisi dans le groupe constitué par les atomes d'halogène, les groupes alkyle inférieurs et le groupe nitro ;
à condition que lorsque 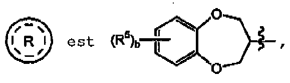 alors a vaille 1 ;
alkyle inférieur désignant une composition de chaîne carbonée constituée de 1-4 atomes de carbone.

4. Composé ou sel pharmaceutiquement acceptable de celui-ci destiné à être utilisé selon la revendication 3, dans lequel
R¹ et R² sont chacun indépendamment choisis dans le groupe constitué par l'atome d'hydrogène et les groupes alkyle inférieurs ;
R⁴ est choisi dans le groupe constitué par l'atome d'hydrogène et le groupe méthyle ;
a est un nombre entier de 1 à 2 ; 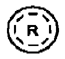 est choisi dans le groupe constitué par les groupes 2-(2,3-dihydrobenzo[1,4]dioxinyle), 2-(benzo[1,3]dioxolyle), 2-(3,4-dihydro-2H-benzo[1,4]dioxépinyle), 2-(2,3-dihydrobenzo[1,4]dioxinyle), 2-(6-chloro-2,3-dihydrobenzo[1,4]dioxinyle), 2-(6-fluoro-2,3-dihydrobenzo[1,4]dioxinyle), 2-(chromanyle), 2-(5-fluoro-2,3-dihydrobenzo[1,4]dioxinyle), 2-(7-chloro-2,3-dihydrobenzo[1,4]dioxinyle, 2-(6-chlorobenzo[1,3]dioxolyle), 2-(7-nitro-2,3-dihydrobenzo[1,4]dioxinyle), 2-(7-méthyl-2,3-dihydrobenzo[1,4]dioxinyle), 2-(5-chloro-2,3-dihydrobenzo[1,4]dioxinyle), 2-(6-bromo-2,3-dihydrobenzo[1,4]dioxinyle), 2-(6,7-dichloro-2,3-dihydrobenzo[1,4]dioxinyle), 2-(8-chloro-2,3-dihydrobenzo[1,4]dioxinyle), 2-(2,3-dihydronaphto[2,3-b][1,4]dioxinyle) et 2-(4-méthylbenzo[1,3]dioxolyle) ; à condition que lorsque 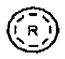 est le groupe 2-(3,4-dihydro-2H-benzo[1,4]dioxépinyle), alors a vaille 1 ; alkyle inférieur désignant une composition de chaîne carbonée constituée de 1-4 atomes de carbone.

5. Composé ou sel pharmaceutiquement acceptable de celui-ci destiné à être utilisé selon la revendication 4, dans lequel
R¹ et R² sont chacun indépendamment choisis dans le groupe constitué par l'atome d'hydrogène et le groupe méthyle ;
R⁴ est choisi dans le groupe constitué par l'atome d'hydrogène et le groupe méthyle ;
a est un nombre entier de 1 à 2 ; 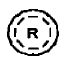 est choisi dans le groupe constitué par les groupes 2-(benzo[1,3]dioxolyle), 2-(2,3-dihydrobenzo[1,4]dioxinyle), 2-(2,3-dihydrobenzo[1,4]dioxinyle), 2-(6-chloro-2,3-dihydrobenzo[1,4]dioxinyle), 2-(7-chloro-2,3-dihydrobenzo[1,4]dioxinyle, 2-(7-méthyl-2,3-dihydrobenzo[1,4]dioxinyle), 2-(6-bromo-2,3-dihydrobenzo[1,4]dioxinyle) et 2-(6,7-dichloro-2,3-dihydrobenzo[1,4]dioxinyle).

6. Composé ou sel pharmaceutiquement acceptable de celui-ci destiné à être utilisé selon la revendication 1, le composé de formule (I) étant choisi dans le groupe constitué par le (2*S*)-(-)-*N*-(6-chloro-2,3-dihydrobenzo[1,4]dioxin-2-ylméthyl)sulfamide ; et les sels pharmaceutiquement acceptables de celui-ci.

7. Composé représenté par la formule (II) 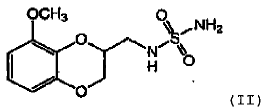 ou sel pharmaceutiquement acceptable de celui-ci destiné à être utilisé dans un procédé de traitement de la migraine.

8. composé destiné à être utilisé selon la revendication 6, le composé de formule (I) étant le (2*S*)-(-)-*N*-(6-chloro-2,3-dihydrobenzo[1,4]dioxin-2-ylméthyl)sulfamide :
